# EUROPEAN PATENT APPLICATION

(11) **EP 3 831 824 A1**
(43) Date of publication of application: **09.06.2021**
(21) Application number: 19844405.1
(22) Date of filing: 31.07.2019
(51) Int. Cl.: C07D 403/02, C07D 209/00, C07D 471/04, C07D 487/04, A61K 31/519, A61K 31/41, A61P 35/00, A61P 37/00, A61P 31/00

(54) **PREPARATION AND APPLICATION OF CLASS OF N-CONTAINING HETEROCYCLIC COMPOUNDS HAVING IMMUNOREGULATORY FUNCTION**

(30) Priority: 01.08.2018 CN 201810866360
(71) Applicant: Shanghai Ennovabio Pharmaceuticals Co., Ltd., Shanghai 201203 (CN)
(72) Inventor: ZHANG, Yi, Shanghai 201203 (CN); DENG, Jianwen, Shanghai 201203 (CN); FENG, Zhiyong, Shanghai 201203 (CN); JIANG, Lei, Shanghai 201203 (CN); LU, Xiaoli, Shanghai 201203 (CN); SHANG, Ke, Shanghai 201203 (CN); SHOU, Jianyong, Shanghai 201203 (CN); WANG, Bing, Shanghai 201203 (CN); XU, Xueli, Shanghai 201203 (CN); XU, Yuan, Shanghai 201203 (CN)
(74) Representative: dompatent von Kreisler Selting Werner - Partnerschaft von Patent- und Rechtsanwälten mbB
(86) International application number: PCT/CN2019/098686
(87) International publication number: WO 2020/024997

(57) **Abstract**

The present invention discloses the preparation and application of heterocyclic compound having immunoregulatory function. Specifically, the present invention discloses a compound having a structure according to Formula I, wherein the definition of each group is as described in the specification. The invention also provides the use of the compounds in regulating immunity and inhibiting PD-1/PD-L1.

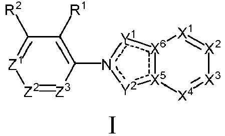

## Description

### FIELD OF THE INVENTION

The present invention relates to the field of small molecule protein inhibitors. Specifically, provided herein are the preparation and application of heterocyclic compounds having immunomodulatory function.

### BACKGROUND OF THE INVENTION

The immune system has functions such as surveillance, defense, and regulation. Cellular immunity is mainly involved in the immune response to intracellular parasitic pathogenic microorganisms and tumor cells, the formation of delayed type hypersensitivity and autoimmune diseases, transplantation rejection and the regulation of humoral immunity. The activation of T lymphocytes by antigen presenting cells is usually regulated by two different signals. Primary signal is transduced by presenting foreign antigen peptides through the major histocompatibility complex (MHC) on APC cells to the T cell receptor (TCR). Secondary signals, also known as costimulatory signals, are transduced through the interaction of costimulatory molecules on APC cells with T cell surface receptors, to regulate the proliferation of T cell, secretion of cytokine and effector functions.Secondary signals include positive regulation and negative regulation. Positive signals promote T cell activation and negative signals induce T cell tolerance, which is essential for the human body to adapt and adjust the response of self-immune cells to different external antigens.

Programmed death-ligand 1 (PD-L1), is also known as cluster of differentiation 274 (CD274) or B7 homolog1 (B7-H1), belongs to tumor necrosis factor superfamily and is a type I transmembrane glycoprotein consisting of 290 amino acid residues. It contains an IgV-like domain, an IgC-like domain, a hydrophobic transmembrane domain, and an intracellular tail containing 30 amino acids. The molecular weight of PD-L1 is 40kDa. PD-L1 mRNA is present in almost all tissues, while PD-L1 protein is only constitutively expressed in a few tissues, including liver, lungs, tonsils, and immune amnesty tissues, such as eyes, placenta, etc. PD-L1 is also expressed on activated T cells, B cells, monocytes, dendritic cells, macrophages, etc.

The receptor of PD-L1 is PD-1, which is mainly expressed on the surface of activated immune cells, such as CD4⁺ T cells, CD8⁺ T cells, NK cells, B cells, monocytes, etc. The binding of PD-L1 to PD-1 can initiate the phosphorylation of tyrosine residues in ITIM (immunoreceptor tyrosine inhibitory motif) in PD-1 cytoplasmic region, promote the binding of tyrosine phospholipase to SHP2, activate SHP2, and dephosphorylate downstream Syk and PI3K, thereby transmitting a termination signal and inhibiting the interaction between antigen-presenting cells or dendritic cells with T cells. Such binding can further inhibit the metabolism of T cells, inhibit the secretion of anti-apoptotic protein Bcl-2, reduce the secretion of effector cytokines IL-2, IFN-γ, and induce T cell depletion and apoptosis, thereby reducing immune responses in which immune T cells are involved, and exerting negative regulation.

After T cells recognize the antigen and are activated, IFN-γ will be secreted. T cell-derived IFN-γ will expand T cells and maintain functions of T cells, such as up-regulating MHC molecules, enhancing antigen processing and presentation of target cells, and promoting T cell differentiation. IFN-γ will also induce the expression of PD-L1 at the site of immune inflammation in a tissue, thereby preventing the tissue from being damaged by excessive immunity. IFN-γ can induce the expression of PD-L1 on the surface of normal epithelial cells, vascular endothelial cells, myeloid cells, naive T cells, and the like. IFN-γ-regulatory factor 1 (IRF-1) induced by interferon can also bind to interferon regulatory factor binding sites at 200bp and 320bp upstream to the transcription start site of PD-L1, thereby regulating PD-L1 at the transcription level. PD-L1 can bind PD-1 on the surface of T cells to exert negative regulation, thereby protecting inflammatory sites.

The negative regulation of PD-L1 plays an important role in tumor immunology. In 2004, Konishi et al. first found that PD-L1 was expressed in tissue samples from patients with non-small cell lung cancer, and then PD-L1 was found to be expressed in the tissues of patients with various tumors, including gastric cancer, lung cancer, liver cancer, and intrahepatic cholangiocarcinoma, colon cancer, pancreatic cancer, ovarian cancer, breast cancer, cervical cancer, head and neck squamous cell carcinoma, nasopharyngeal carcinoma, esophageal cancer, bladder cancer, renal cell carcinoma, skin cancer, oral squamous cell carcinoma, etc. During the malignant transformation of cells, new protein molecules will be generated due to gene mutations, exogenous gene (viral) expression or static gene activation, and the like. After these new proteins are degraded in a cell, certain degraded peptide fragments can be expressed on the cell surface and become tumor antigens. The immune system can recognize tumor antigens and eliminate tumor cells through immune monitoring, while tumor cells can escape immune attacks by means of PD-L1.

The expression of PD-L1 at the tumor site can protect tumor cells through various ways. Tumor infiltrating lymphocytes (TIL) secretes IFN-γ, which can induce tumor cells and surrounding stromal cells to express PD-L1. PD-L1 of tumor cells can bind to PD-1 on TIL, inhibit the activation of TIL cells, and further cause apoptosis thereof. *In vitro* experiments have shown that tumor cell-associated PD-L1 can increase the apoptosis of tumor-specific T cells, while PD-L1 monoclonal antibodies can reduce such effect. Tumor-associated PD-L1 can promote the expression of IL-10 by T cells, and further inhibit the immune response. PD-L1 is not only a ligand of PD-1, but also can act as a receptor to transmit reverse signals to protect tumor cells from apoptosis induced by other anti-tumor pathways, such as FAS-FASL.

Several marketed monoclonal antibody drugs targeting PD-1 or PD-L1 have proven that blockers for PD-1 / PD-L1 can be clinically useful in the treatment of various tumors. However, antibody drugs exhibit their own characteristics, such as high production cost, poor stability, necessity to be administered by injection, and proneness to inducing immunogenicity, etc. Small molecule drugs have advantages, such as good tissue permeability, convenient storage and transportation, low production cost, non-immunogenicity, and availability of oral administration, etc. Therefore, it is of use and social significance to research and develop small molecule blockers for PD-1 / PD-L1.

### SUMMARY OF THE INVENTION

The purpose of the present invention is to provide a class of small molecule PD-1/PD-L1 blockers.

The first aspect of the present invention provides a compound according to Formula I, the stereoisomers or tautomers thereof, or pharmaceutically acceptable salts, hydrates or solvates thereof: 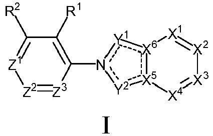
X¹, X², X³ and X⁴ are each independently selected from the group consisting of N and CR³;
X⁵ and X⁶ are each independently selected from the group consisting of N and C;
Y¹ and Y² are each independently N, C, C=O, S(=O), S(=O)₂, O or S;
Z¹, Z² and Z³ are each independently N or CR⁴;
R¹ is selected from the group consisting of H, halogen, CN, substituted or unsubstituted C₁-C₆ alkyl, substituted or unsubstituted C₁-C₆ alkoxyl;
R² is selected from the group consisting of substituted or unsubstituted C₆-C₁₀ aryl, substituted or unsubstituted 5-10 membered heteroaryl having 1-3 heteroatoms selected from the group consisting of N, S and O, or substituted or unsubstituted 5-10 membered heterocyclyl with 1-3 heteroatoms selected from the group consisting of N, S and O; and one or more H on R^{Z} are replaced by R⁵;
R³ is selected from the group consisting of H, substituted or unsubstituted C₁-C₆ alkyl, substituted or unsubstituted C₁-C₆ alkoxyl, 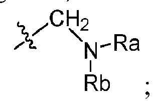 and at least one R³ is 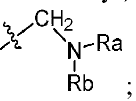
Ra and Rb are independently selected from H, -(C=O)-substituted or unsubstituted C₁-C₈ alkyl, substituted or unsubstituted C₁-C₈ alkyl, substituted or unsubstituted C₂-C₆ alkenyl, substituted or unsubstituted C₂-C₆ alkynyl, substituted or unsubstituted C₁-C₈ alkylamino, substituted or unsubstituted C₁-C₈ alkoxyl, substituted or unsubstituted C₃-C₁₀ cycloalkyl, substituted or unsubstituted 3-10 membered heterocyclyl with 1-3 heteroatoms selected from the group consisting of N, S and O, substituted or unsubstituted C₆-C₁₀ aryl, substituted or unsubstituted 5-10 membered heteroaryl with 1-3 heteroatoms selected from the group consisting of N, S and O, or substituted or unsubstituted 5-10 membered heterocyclyl with 1-3 heteroatoms selected from the group consisting of N, S and O; or
Ra and Rb and adjacent N atoms together form a substituted or unsubstituted 5-10 membered heterocyclyl with 1-3 heteroatoms selected from the group consisting of N, S and O;
R⁴ is selected from the group consisting of H, halogen, CN, substituted or unsubstituted C₁-C₆ alkyl, substituted or unsubstituted C₁-C₆ alkoxyl;
R⁵ is selected from the group consisting of H, CN, halogen, substituted or unsubstituted-L₁-L₂(C₁-C₈ alkyl)-O-(C₁-C₈ alkylene)-(substituted or unsubstituted 5 -7 membered heteroaryl), substituted or unsubstituted -L₁-L₂(C₁-C₈ alkyl)-O-(C₁-C₈ alkylene)-N(Ra)(Rb), -O-substituted or unsubstituted -(C₁-C₈ alkylene)-O-(C₁-C₈ alkylene)-N(Ra)(Rb), or -NH-C(=O)-(substituted or unsubstituted 5-7 membered heteroaryl); wherein L₁ and L₂ are each independently selected from the group consisting of none, substituted or unsubstituted C₁-C₈ alkylene, -NH-C(=O)-NH-, -C (=O)-NH-, -NH-C(=O)-, -C(=O)-, -O-, -S- or -NH-; or two R⁵ and connected carbon atoms together form a group selected from substituted or unsubstituted 5-7 membered heteroaryl, substituted or unsubstituted 5-7 membered heterocyclyl;
unless otherwise specified, "substituted" refers to being replaced by one or more (for example, 2, 3, 4, etc.) substituents selected from the group consisting of carboxyl, halogen, C₁-C₆ alkoxyl, halogenated C₁-C₆ alkoxyl, C₃-C₈ cycloalkyl, halogenated C₃-C₈ cycloalkyl, methyl sulfone group, -S(=O)₂NH₂, oxo(= O), -CN, hydroxyl, -NH₂, C₁-C₆ amine, C₁-C₆ amide (-C(=O)-N(Rc)₂ or -NH-C(=O)(Rc), Rc is H or C₁-C₅ alkyl), 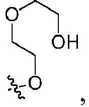 or substituted or unsubstituted groups selected from the group consisting of C₁-C₆ alkyl, C₆-C₁₀ aryl, 5-10 membered heteroaryl with 1-3 heteroatoms selected from N, S and O, -(CH₂)-C₆-C₁₀ aryl, -(CH₂)- (5-10 membered heteroaryl with 1-3 heteroatoms selected from N, S and O), and substituents selected from the group consisting of halogen, C₁-C₆ alkyl, C₁-C₆ alkoxyl, oxo, -CN, -OH, C₆-C₁₀ aryl, 5-10 membered heteroaryl with 1-3 heteroatoms selected from N, S and O; 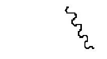 is the attachment site of the group;
each 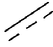 is a single bond or a double bond independently;
with the proviso that the compound of Formula I has a chemically stable structure.

In another preferred embodiment, the compound has a structure according to Formula II-1 or Formula II-2:

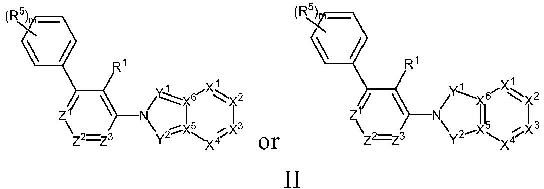

In another preferred embodiment, R² is selected from the group consisting of

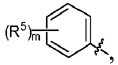

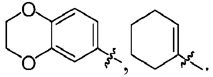

In another preferred embodiment, R² is selected from the group consisting of substituted or unsubstituted phenyl.

In another preferred embodiment, 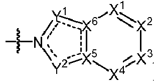 has a structure selected from the following group:

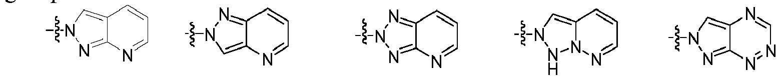

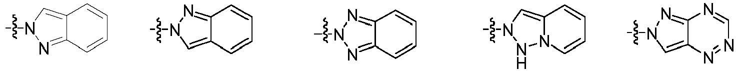

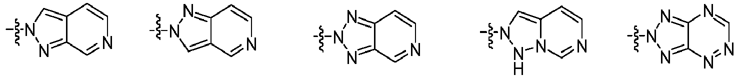

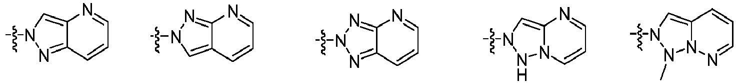

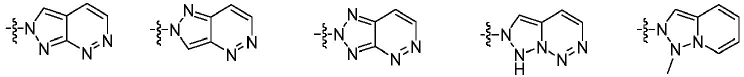

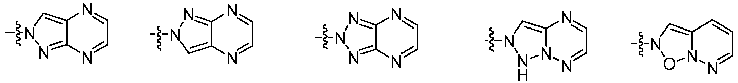

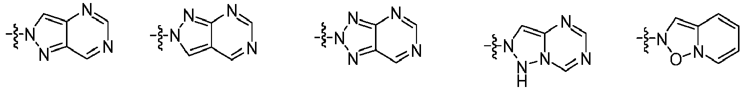

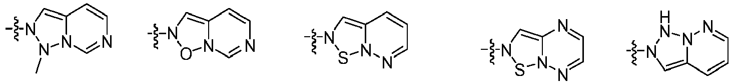

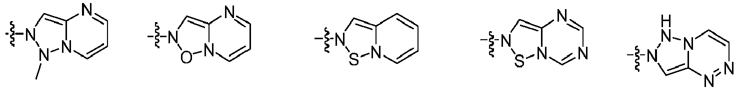

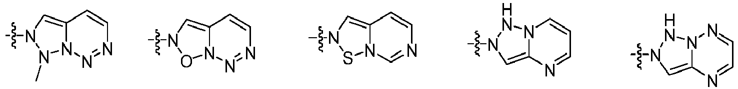

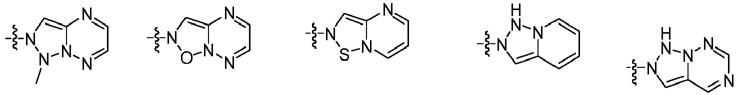

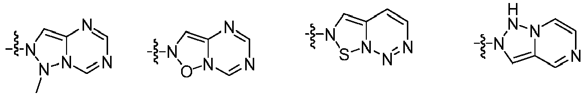

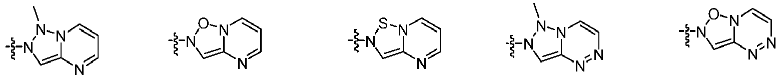

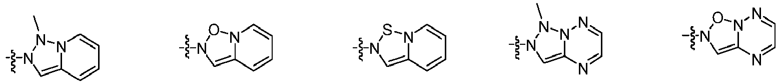

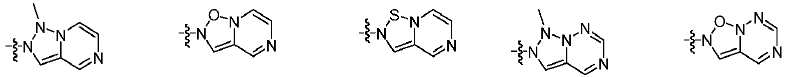

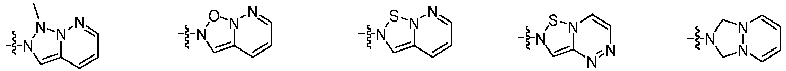

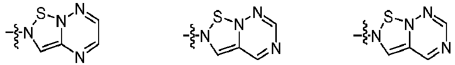

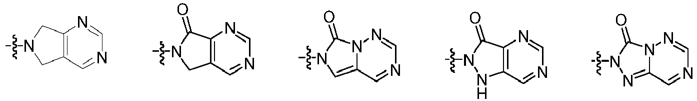

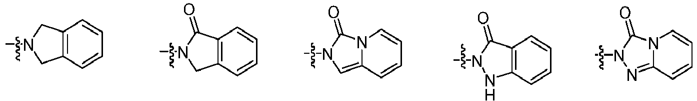

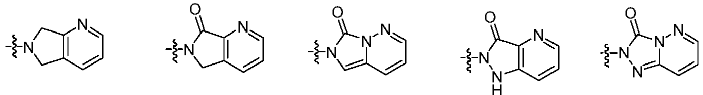

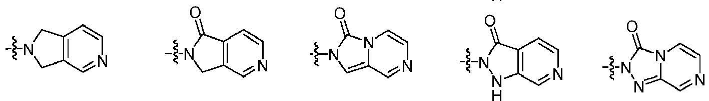

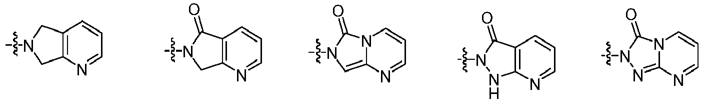

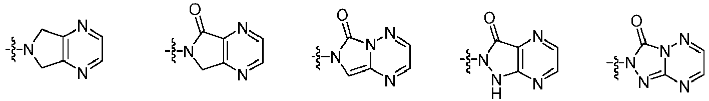

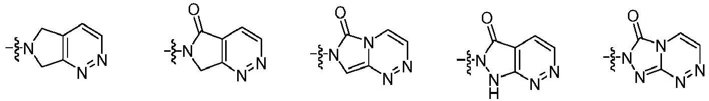

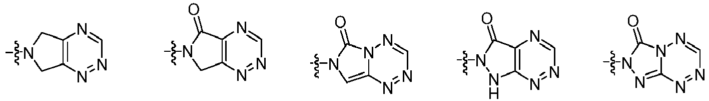

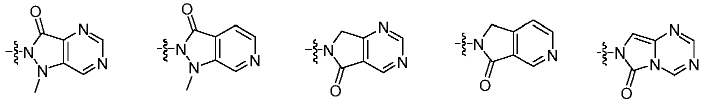

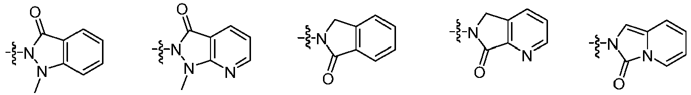

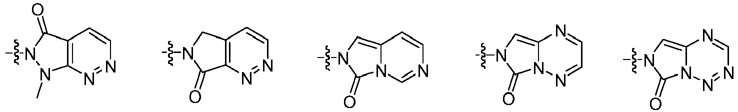

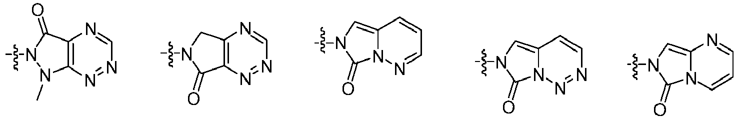

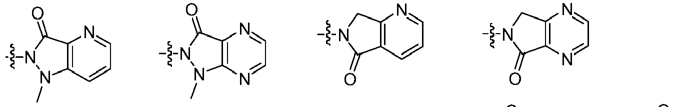

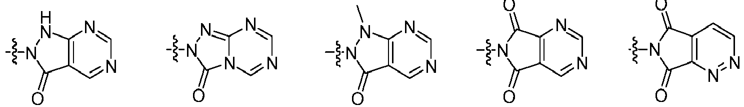

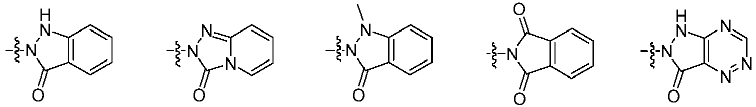

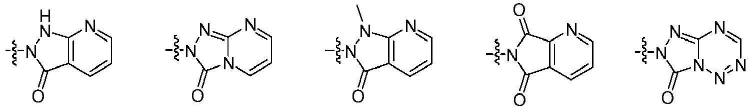

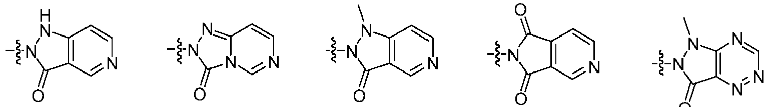

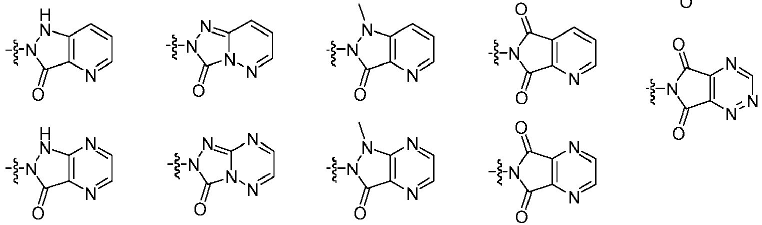

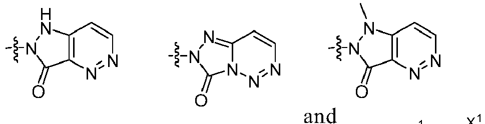

In another preferred embodiment, 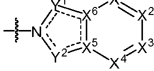 is a structure formed by heterocycle selected from the group consisting of:

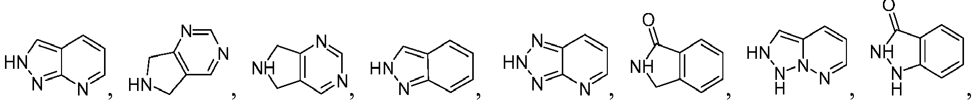

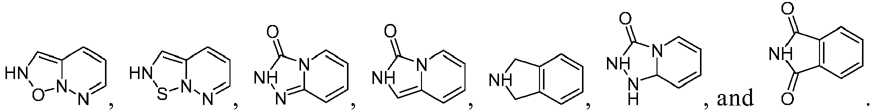

In another preferred embodiment, R³ is -CH₂-R, wherein R is selected from the group consisting of:

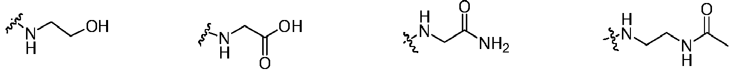

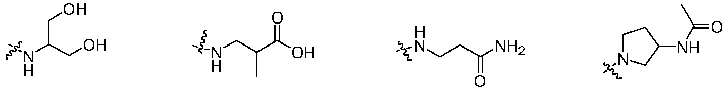

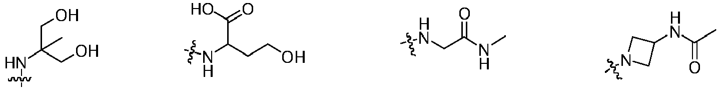

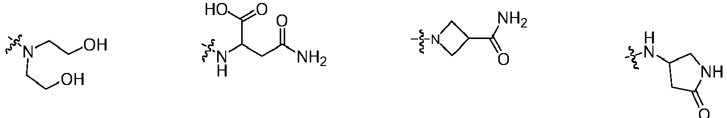

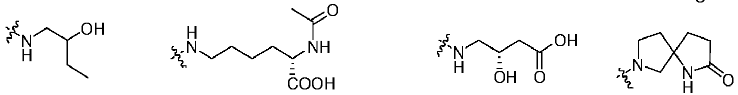

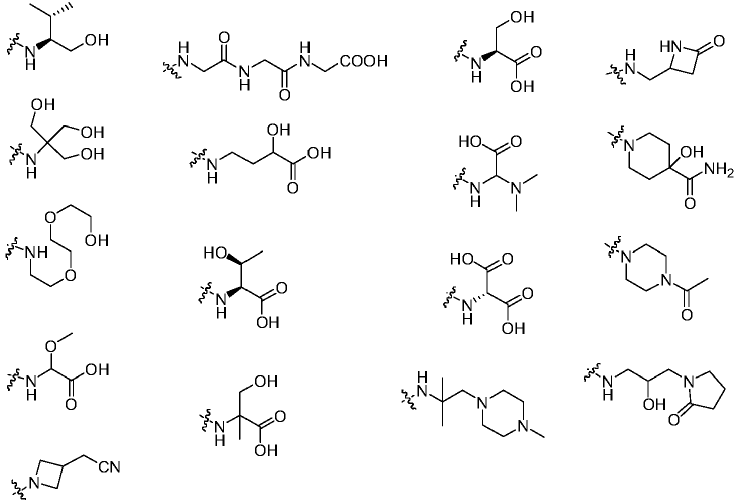

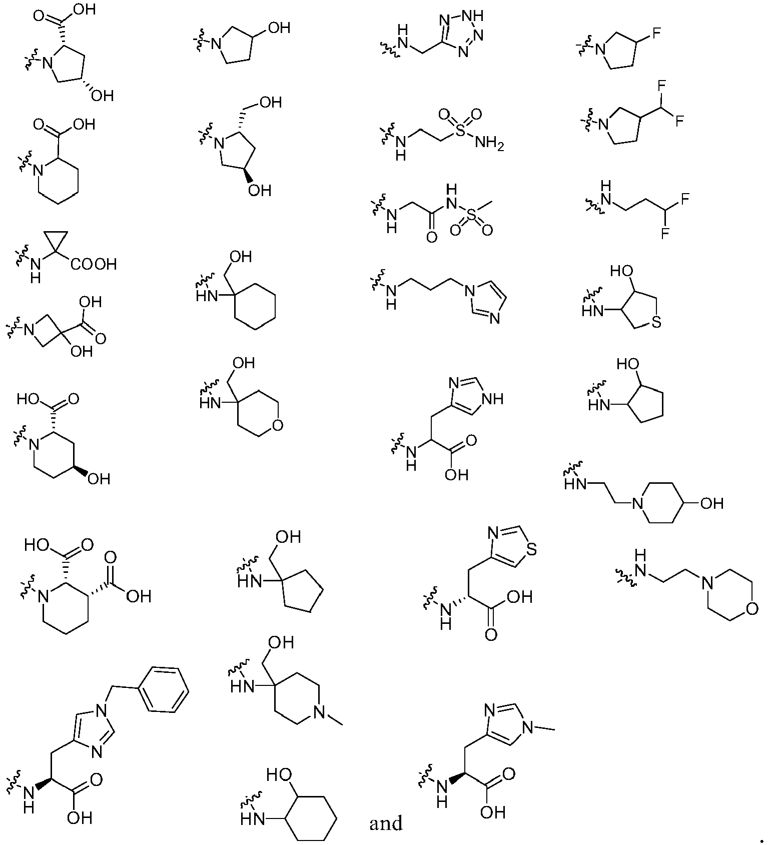

In another preferred embodiment, R⁵ is selected from the group consisting of H, Me, Cl, CN, -O(CH₂)ₙ- (substituted or unsubstituted 5-7 membered heteroaryl), -O(CH₂)ₙ-N(Ra)(Rb).

In another preferred embodiment, R⁵ is selected from the group consisting of H, Me, Cl, CN, or a group selected from the group consisting of: 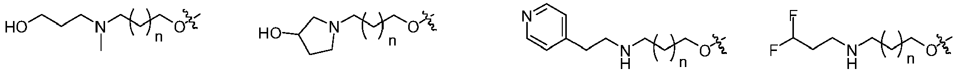 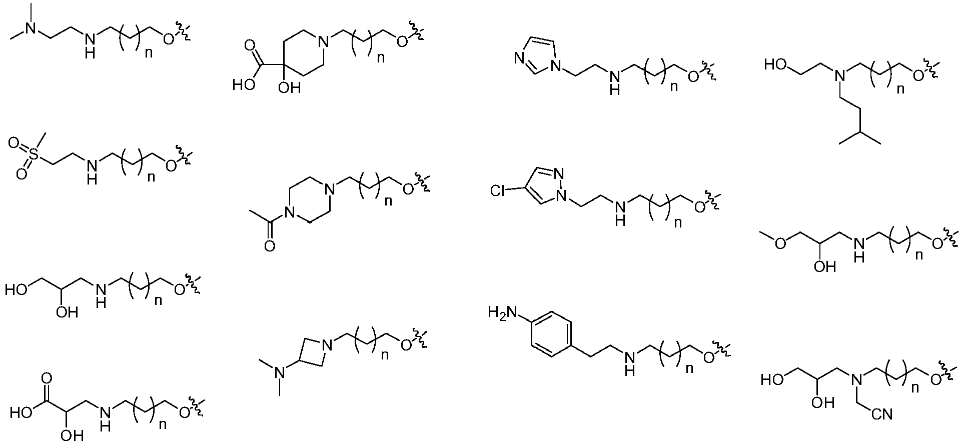 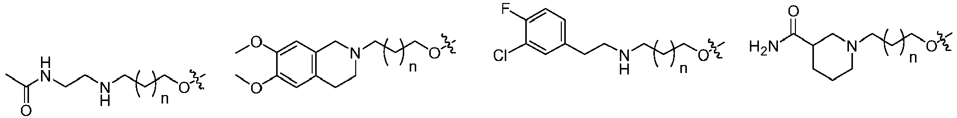 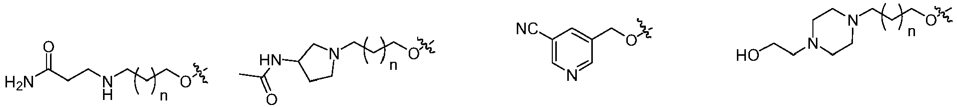 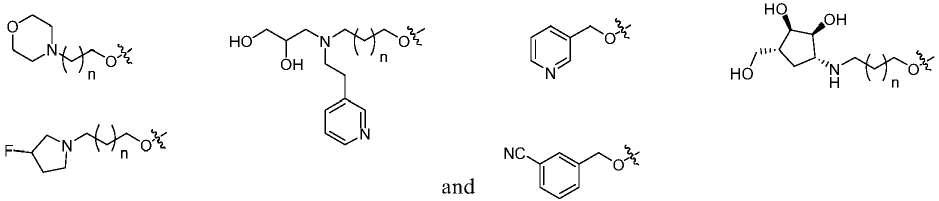 wherein n=1-2.

In another preferred embodiment, the compound of Formula I is selected from the group consisting of:

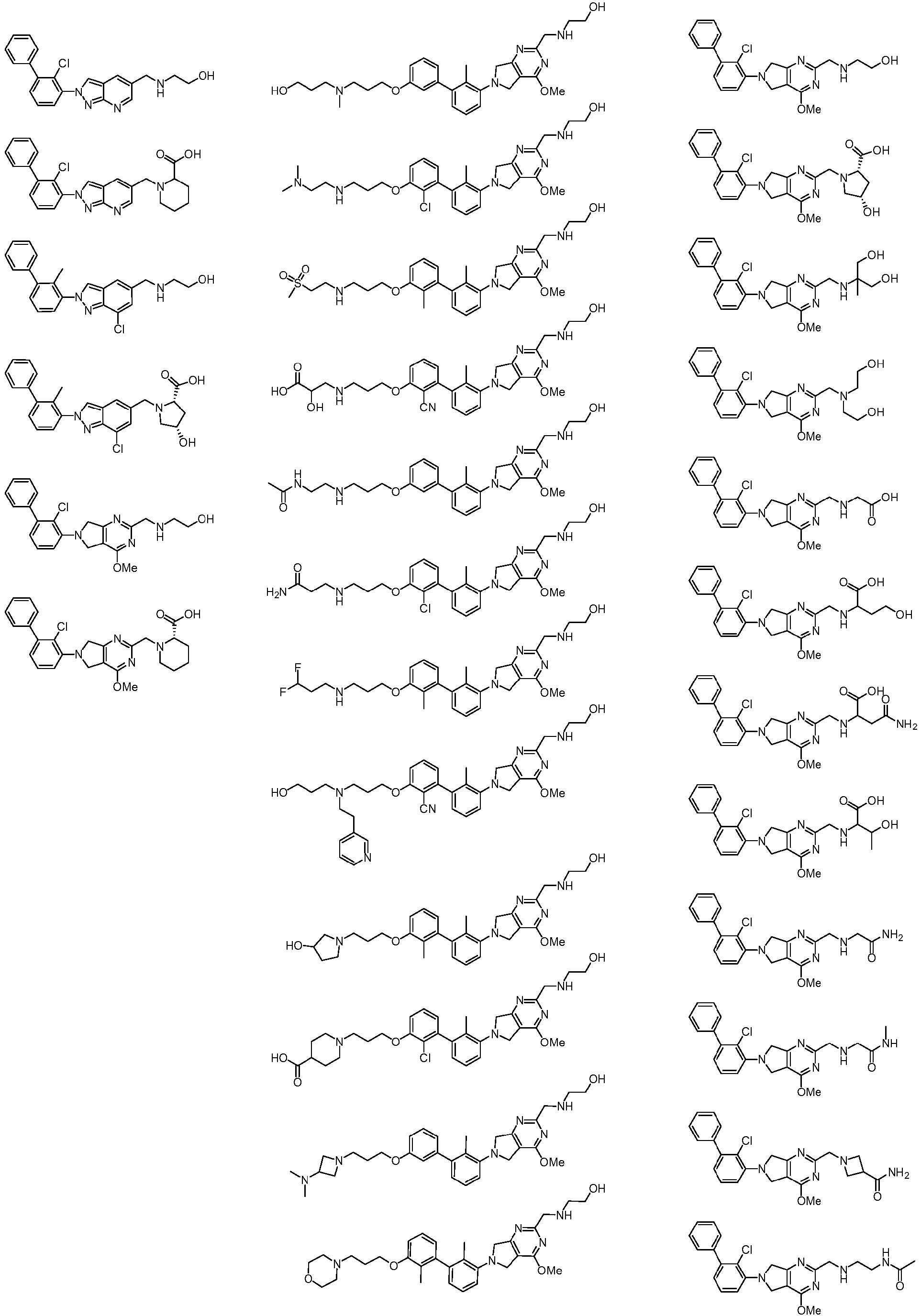

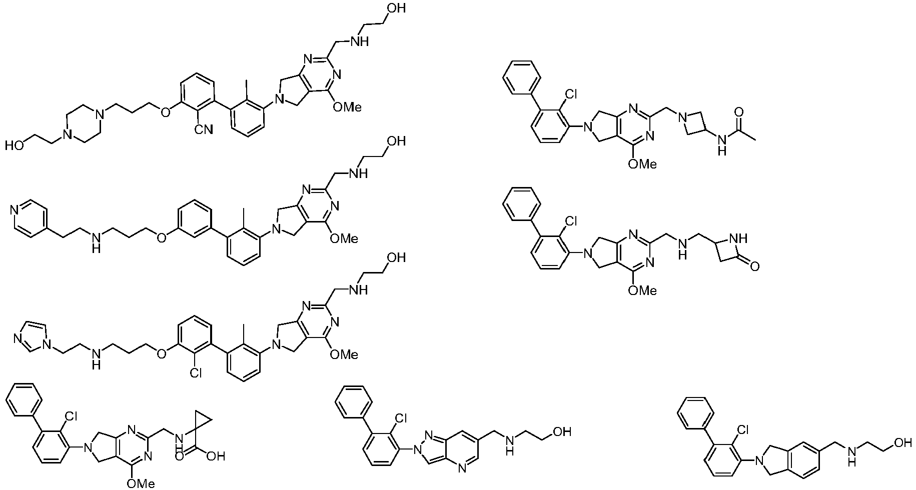

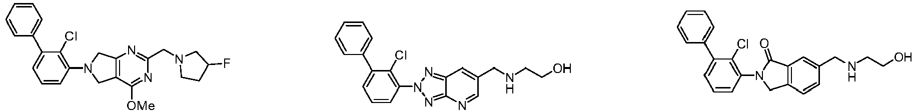

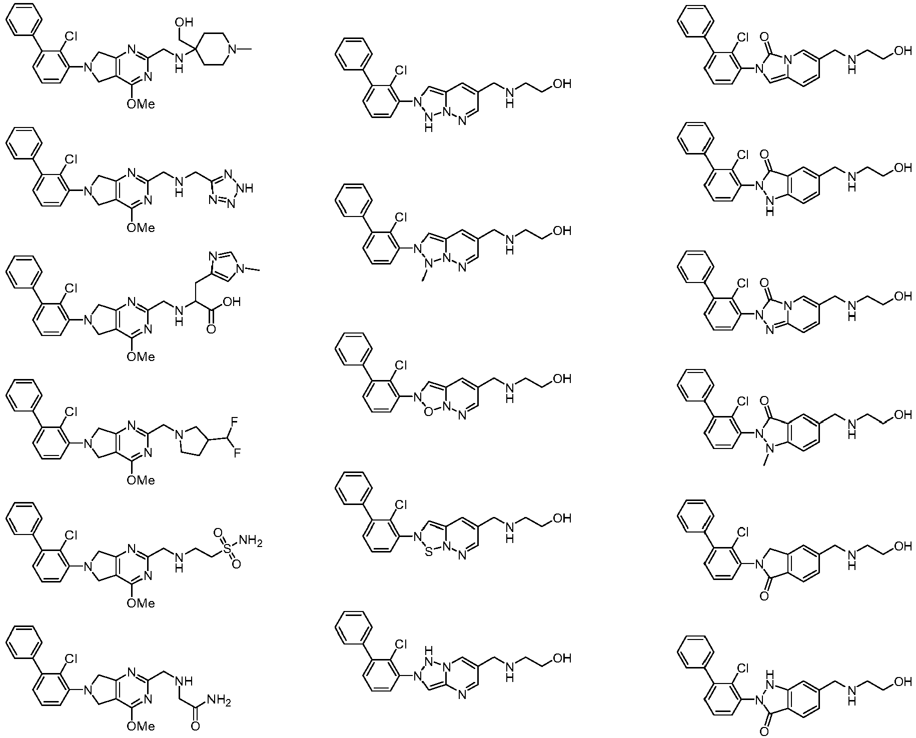

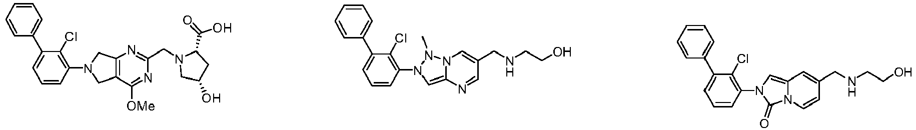

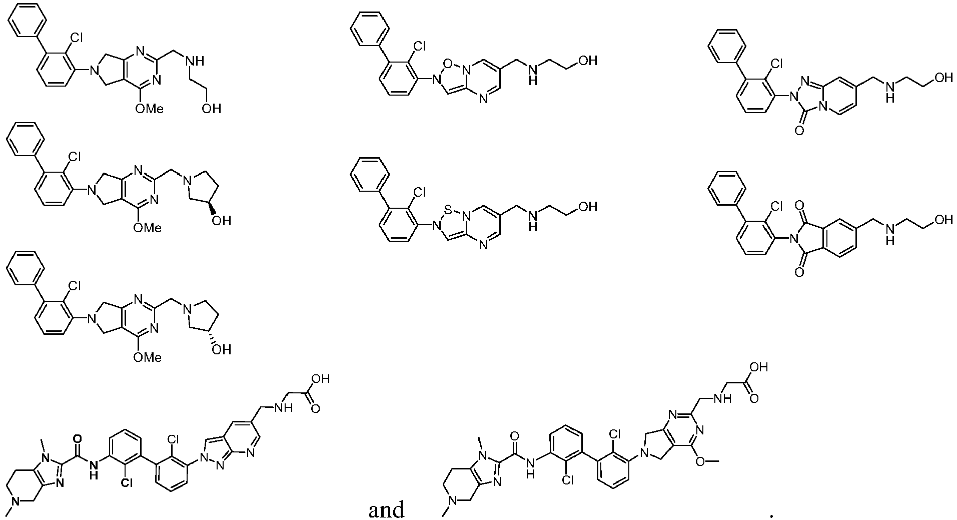

In another preferred embodiment, the compound of Formula I is selected from the group consisting of:

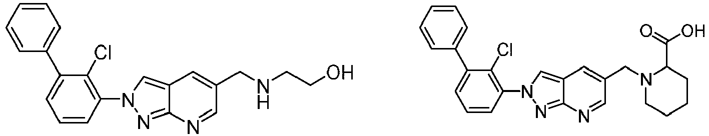

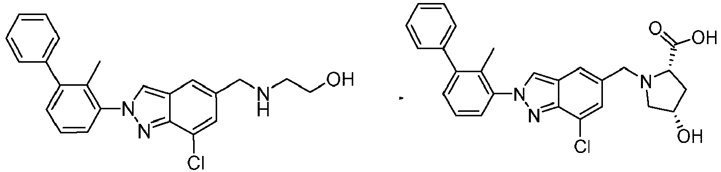

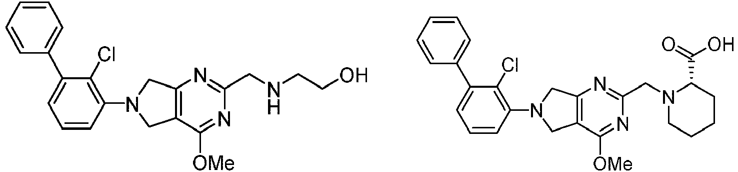

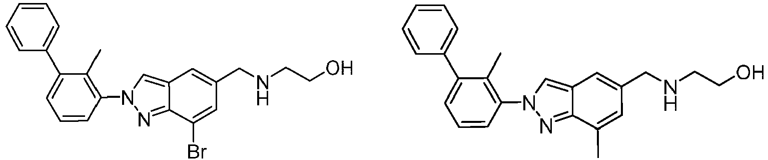

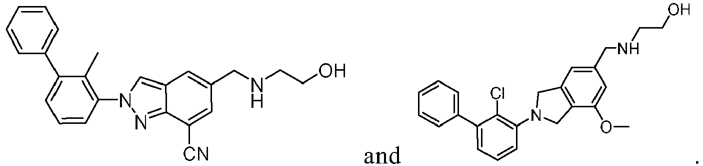

In the second aspect of the present invention, a pharmaceutical composition which comprises (1) the compound, or stereoisomers or tautomers thereof, or pharmaceutically acceptable salts, hydrates or solvates thereof according to the first aspect of the present invention; (2) a pharmaceutically acceptable carrier is provided.

The third aspect of the present invention provides the use of the compounds, or stereoisomers or tautomers thereof, or pharmaceutically acceptable salts, hydrates or solvates thereof according to the first aspect of the present invention, or the pharmaceutical composition according to the second aspect of the present invention for preparing pharmaceutical compositions for preventing and/or treating diseases related to the activity or expression of PD-1/PD-L1. In some embodiments, the disease is selected from the group consisting of tumors, pathogen infections, and diseases related to autoimmune responses .

In some embodiments, the pharmaceutical composition is used for the treatment of diseases selected from the group consisting of melanoma (e.g. metastatic malignant melanoma), renal cancer (e.g. clear cell carcinoma), prostate cancer (e.g. hormone refractory prostate adenocarcinoma), breast cancer, colon cancer and lung cancer (such as non-small cell lung cancer), bone cancer, pancreatic cancer, skin cancer, head or neck cancer, skin or intraocular melanoma, uterine cancer, ovarian cancer, rectal cancer, anal cancer, gastrointestinal cancer, testicular cancer, uterine cancer, fallopian tube cancer, endometrial cancer, cervical cancer, vaginal cancer, vulva cancer, Hodgkin's disease, non-Hodgkin's lymphoma, esophageal cancer, small bowel cancer, endocrine system cancer, thyroid cancer, parathyroid cancer, adrenal cancer, soft tissue sarcoma, urethral cancer, penile cancer, chronic or acute leukemia (including acute myeloid leukemia, chronic myeloid leukemia, acute lymphoblastic leukemia, chronic lymphocytic leukemia), childhood solid tumors, lymphocytic lymphoma tumors, bladder cancer, kidney or ureter cancer, renal pelvis cancer, central nervous system (CNS) neoplasms/tumors, primary CNS lymphoma, tumor angiogenesis, spinal axis tumors, brainstem glioma, pituitary gland adenoma, Kaposi's sarcoma, epidermoid carcinoma, squamous cell carcinoma, T-cell lymphoma, environmentally induced cancers (including those induced by asbestos), and the combinations thereof. Metastatic cancer, especially metastatic cancer that expresses PD-L1.

In some embodiments, the pharmaceutical composition can be used in combination regimen. Preferably, the combination regimen: combined tumor chemotherapy, other tumor immunotherapeutics (small molecule compounds, antibodies, etc.), radiotherapy, tumor targeted therapeutics, and tumor vaccines (such as human papilloma virus (HPV), hepatitis virus (HBV and HCV) and Kaposi herpes sarcoma virus (KHSV).

In some embodiments, the pharmaceutical composition is used alone or in combination for the treatment of patients exposed to specific toxins or pathogens, which includes, but is not limited to, the treatment of various viruses, pathogenic bacteria, pathogenic fungi, pathogenic parasites, etc, e.g., infections established by pathogens, such as HIV, hepatitis virus (A, B, C), influenza virus, herpes virus, Giardia, malaria, Leishmania, *Staphylococcus aureus, Pseudomonas aeruginosa*.In some embodiments, the pharmaceutical composition is used to induce therapeutic autoimmune response.

In some embodiments, the pharmaceutical composition is used to treat patients with inappropriate accumulation of other autoantigens, such as amyloid deposits, including Aβ in Alzheimer's disease, cytokines such as TNFα and IgE .

The fourth aspect of the present invention provides the PD-1/PD-L1 inhibitor, comprising the compound as described in the first aspect of the present invention, or stereoisomers or tautomers thereof, or pharmaceutically acceptable salts, hydrates or solvates thereof.

It should be understood that, in the present invention, each of the technical features specifically described above and below (such as those in the Examples) can be combined with each other, thereby constituting new or preferred technical solutions which need not be specified again herein .

### EMBODIMENTS FOR CARRYING OUT THE INVENTION

The inventor designed and synthesized a novel PD-1 small molecule inhibitor after long-term and in-depth research. The inventor has completed the present invention on this basis.

### Terms

Unless otherwise defined, all technical and scientific terms used herein have the same meaning as commonly understood by one of ordinary skill in the art to which this invention belongs.

As used herein, when used in reference to a particular recited value, the term "about" means that the value can vary by no more than 1% from the recited value. For example, as used herein, the expression "about 100" includes all the values between 99 and 101 and (eg, 99.1, 99.2, 99.3, 99.4, etc.).

As used herein, the terms "containing" or "including (comprising)" may be opened form, semi-closed form, or closed form. In other words, the terms also include situations such as "essentially consisting of..." or "consisting of..."

### Definitions

As used herein, the term "alkyl" includes straight or branched alkyl groups. For example, C₁-C₈ alkyl refers to straight or branched alkyls having from 1-8 carbon atoms, such as methyl, ethyl, propyl, isopropyl, butyl, isobutyl, tert-butyl, and the like.

As used herein, the term "alkenyl" includes straight or branched alkenyl groups. For example, C₂-C₆ alkenyl refers to straight or branched alkenyl groups having 2-6 carbon atoms, such as vinyl, allyl, 1-propenyl, isopropenyl, 1-butenyl, 2-butenyl, and the like.

As used herein, the term "alkynyl" includes straight or branched alkynyl groups. For example, "C₂-C₆ alkynyl" refers to straight or branched alkynyls having 2-6 carbon atoms, such as ethynyl, propynyl, butynyl, and the like.

As used herein, the term "C₃-C₈ cycloalkyl" refers to cycloalkyl groups having 3 to 10 carbon atoms. It may be a monocyclic ring, such as cyclopropyl, cyclobutyl, cyclopentyl, cyclohexyl, and the like. It may also be of bicyclic form, such as bridged or spiro ring form.

As used herein, the term "C₁-C₈ alkoxyl" refers to straight or branched alkoxy groups having 1-8 carbon atoms; for example, methoxy, ethoxy, propoxy, isopropoxy, butoxy, isobutoxy, tert-butoxy, and the like.

As used herein, the term "3-10 membered heterocycloalkyl having 1-3 heteroatoms selected from the group consisting of N, S and O" refers to a saturated or partially saturated cyclic group having 3-10 atoms, wherein 1-3 atoms are heteroatoms selected from the group consisting of N, S and O. It may be a monocyclic ring or bicyclic form, such as bridged or spiro ring form. Specific examples may be oxetane, azetidine, tetrahydro-2H-pyranyl, piperidinyl, tetrahydrofuranyl, morpholinyl and pyrrolidinyl, and the like.

As used herein, the term "C₆-C₁₀ aryl" refers to aryl groups having 6 to 10 carbon atoms, such as phenyl, naphthyl, and the like.

As used herein, the term "5-10 membered heteroaryl having 1-3 heteroatoms selected from the group consisitng of N, S and O" refers to cyclic aromatic groups having 5-10 atoms, of which 1-3 is selected from the group consisting of N, S and O. It may be a monocyclic ring or fused ring form. Specific examples may be pyridyl, pyridazinyl, pyrimidinyl, pyrazinyl, triazinyl, pyrrolyl, pyrazolyl, imidazolyl, (1,2,3)-triazolyl and (1,2,4)-triazolyl, tetrazyl, furyl, thienyl, isoxazolyl, thiazolyl, oxazolyl, etc.

Unless otherwise specified, all the groups described in the present invention may be substituted with substituents selected from the group consisting of halogen, nitrile, nitro, hydroxyl, amino, C₁-C₆ alkyl-amine, C₁-C₆ alkyl, C₂-C₆ alkenyl, C₂-C₆ alkynyl, C₁-C₆ alkoxyl, halogenated C₁-C₆ alkyl, halogenated C₂-C₆ alkenyl, halogenated C₂-C₆ alkynyl, halogenated C₁-C₆ alkoxyl, allyl, benzyl, C₆-C₁₂ aryl, C₁-C₆ alkoxy-C₁-C₆ alkyl, C₁-C₆ alkoxy-carbonyl, phenoxycarbonyl, C₂-C₆ alkynyl-carbonyl, C₂-C₆ alkenyl-carbonyl, C₃-C₆ cycloalkyl-carbonyl, C₁-C₆ alkyl-sulfonyl, etc.

As used herein, "halogen" or "halogen atom" refers to F, Cl, Br, and I. As used herein, "halogen" or "halogen atom" refers to F, Cl, Br, and I. More preferably, the halogen or halogen atom is selected from F, Cl and Br. "Halogenated" means substituted by an atom selected from F, Cl, Br, and I.

Unless otherwise specified, the structural formula described herein are intended to include all isomeric forms (such as enantiomeric, diastereomeric, and geometric isomers (or conformational isomers)): for example, R, S configuration of asymmetrical centers, (Z), (E) isomers of double bonds, etc. Therefore, the single stereochemical isomers or enantiomers, diastereomers or geometric isomers (or conformers) of the compounds of the invention, or mixtures thereof all fall within the scope of the invention.

As used herein, the term "tautomer" means that structural isomers having different energies can exceed the low energy barrier and thereby transform between each other. For example, proton tautomers (proton shift) includes interconversion by proton transfer, such as 1H-carbazole and 2H-carbazole. Valence tautomers include interconversion through some bonding electron recombination.

As used herein, the term "solvate" refers to a complex of specific ratio formed by a compound of the invention coordinating to a solvent molecule.

### Compound of Formula I

Provided herein is a compound according to Formula I, the stereoisomers or tautomers thereof, or pharmaceutically acceptable salts, hydrates or solvates thereof: 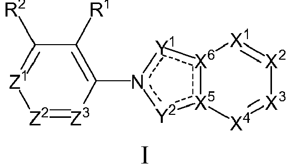
X¹, X², X³ and X⁴ are each independently selected from the group consisting of N and CR³;
X⁵ and X⁶ are each independently selected from the group consisting of N and C;
Y¹ and Y² are each independently N, C, C=O, S(=O), S(=O)₂, O or S;
Z¹, Z² and Z³ are each independently N or CR⁴;
R¹ is selected from the group consisting of H, halogen, CN, substituted or unsubstituted C₁-C₆ alkyl, and substituted or unsubstituted C₁-C₆ alkoxyl;
R² is selected from the group consisting of substituted or unsubstituted C₆-C₁₀ aryl, substituted or unsubstituted 5-10 membered heteroaryl having 1-3 heteroatoms selected from the group consisting of N, S and O, or substituted or unsubstituted 5-10 membered heterocyclyl with 1-3 heteroatoms selected from the group consisting of N, S and O; and one or more H on R² are replaced by R⁵;
R³ is selected from the group consisting of H, substituted or unsubstituted C₁-C₆ alkyl, substituted or unsubstituted C₁-C₆ alkoxyl, 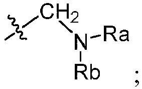 and at least one R³ is 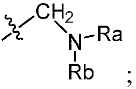
Ra and Rb are independently selected from H, carbonyl, -(C=O)-substituted or unsubstituted C₁-C₈ alkyl, substituted or unsubstituted C₁-C₈ alkyl, substituted or unsubstituted C₂-C₆ alkenyl, substituted or unsubstituted C₂-C₆ alkynyl, substituted or unsubstituted C₁-C₈ alkylamino, substituted or unsubstituted C₁-C₈ alkoxyl, substituted or unsubstituted C₃-C₁₀ cycloalkyl, substituted or unsubstituted 3-10 membered heterocyclyl with 1-3 heteroatoms selected from the group consisting of N, S and O, substituted or unsubstituted C₆-C₁₀ aryl, substituted or unsubstituted 5-10 membered heteroaryl with 1-3 heteroatoms selected from the group consisting of N, S and O, or substituted or unsubstituted 5-10 membered heterocyclyl with 1-3 heteroatoms selected from the group consisting of N, S and O; or
Ra and Rb and adjacent N atoms together form a substituted or unsubstituted 5-10 membered heterocyclyl with 1-3 heteroatoms selected from the group consisting of N, S and O;
R⁴ is selected from the group consisting of H, halogen, CN, substituted or unsubstituted C₁-C₆ alkyl, substituted or unsubstituted C₁-C₆ alkoxyl;
R⁵ is selected from the group consisting of H, CN, halogen, substituted or unsubstituted-L₁-L₂(C₁-C₈ alkyl)-O-(C₁-C₈ alkylene alkyl)-(substituted or unsubstituted 5 -7 membered heteroaryl), substituted or unsubstituted -L₁-L₂(C₁-C₈ alkyl)-O-(C₁-C₈ alkylene alkyl)-N(Ra)(Rb), -O-substituted or unsubstituted -(C₁-C₈ alkylene alkyl)-O-(C₁-C₈ alkylene alkyl)-N(Ra)(Rb); wherein L₁ and L₂ are each independently selected from the group consisting ofnone, substituted or unsubstituted C₁-C₈ alkylene, -NH-C(=O)-NH-, -C (=O)-NH-, -NH-C(=O)-, -C(=O)-, -O-, -S- or -NH-; or two R⁵ and connected carbon atoms together form a group selected from substituted or unsubstituted 5-7 membered heteroaryl, substituted or unsubstituted 5-7 membered heterocyclyl;
unless otherwise specified, "substituted" refers to being replaced by one or more (for example, 2, 3, 4, etc.) substituents selected from the group consisting of halogen, C₁-C₆ alkoxyl, halogenated C₁-C₆ alkoxyl, C₃-C₈ cycloalkyl, halogenated C₃-C₈ cycloalkyl, methyl sulfone group, -S(=O)₂NH₂, oxo= O), -CN, hydroxyl, -NH₂, C₁-C₆ amine, carboxyl, C₁-C₆ amide (-C(=O)N(Rc)₂ or -NH-C(=O)(Rc), Rc is H or C₁-C₅ alkyl), 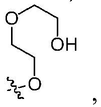 or substituted or unsubstituted groups selected from the group consisting of C₁-C₆ alkyl, C₆-C₁₀ aryl, 5-10 membered heteroaryl wkith 1-3 heteroatoms selected from N, S and O, -(CH₂)-C₆-C₁₀ aryl, -(CH₂)- (5-10 membered heteroaryl with 1-3 heteroatoms selected from N, S and O), and substituents selected from the group consisting of halogen, C₁-C₆ alkyl, C₁-C₆ alkoxyl, -O-, -CN, -OH, C₆-C₁₀ aryl, 5-10 membered heteroaryl with 1-3 heteroatoms selected from N, S and O; 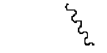 is the attachment site of the group;
each 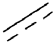 is a single bond or a double bond independently;
with the proviso that the compound of Formula I has a chemically stable structure.

The preferred compound of Formula I is the specific compound shown in the example of the present application.

### Preparation of Compound of Formula I

The invention also provides a method for preparing the compound as described in the first aspect of the invention which includes the steps of (1) or (2):

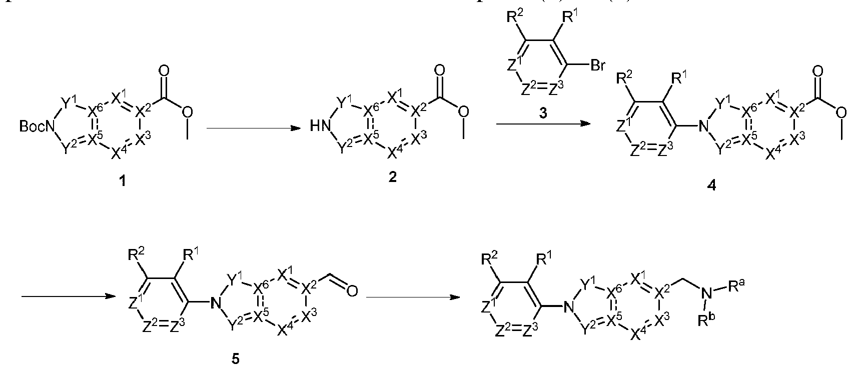

The Boc protecting group of compound 1 is removed under acidic environment to obtain compound 2, and then reacted with compound 3 with the presence of palladium catalyst, alkali and phosphine compound to obtain compound 4. Compound 4 is reduced to obtain compound 5, and then compound 5 is subjected to reductive amination reaction to obtain the compound of Formula I.

### Pharmaceutical Composition and Administration Thereof

Since the compound herein has excellent PD-1 inhibitory activity, the compound and various crystal forms thereof, pharmaceutically acceptable inorganic or organic salts, hydrates or solvates thereof herein, and pharmaceutical composition containing the compound according to the present invention as main active ingredient can be used to prevent and/or treat diseases related to the PD-1/PD-L1 signaling pathway (for example, cancer).

The pharmaceutical composition of the invention comprises the compound of the present invention in a safe and effective dosage range and pharmaceutically acceptable excipients or carriers. Wherein the "safe and effective dosage" means that the amount of compound is sufficient to significantly ameliorate the condition without causing significant side effects. Generally, the pharmaceutical composition contains 1-2000 mg polymorphs of the invention per dose, preferably, 10-200 mg polymorphs of the invention per dose. Preferably, the "dose" is a capsule or tablet.

"Pharmaceutically acceptable carrier" means one or more compatible solid or liquid fillers, or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween®), wetting agent (such as sodium dodecyl sulfate), coloring agents, flavoring agents, stabilizers, antioxidants, preservatives, pyrogen-free water, etc.

There is no special limitation of administration mode for the compound or pharmaceutical compositions of the present invention, and the representative administration mode includes (but is not limited to): oral, parenteral (intravenous, intramuscular or subcutaneous).

Solid dosage forms for oral administration include capsules, tablets, pills, powders and granules. In these solid dosage forms, the active compounds are mixed with at least one conventional inert excipient (or carrier), such as sodium citrate or CaHPO4, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or the mixtures thereof. In capsules, tablets and pills, the dosage forms may also contain buffering agents.

The solid dosage forms such as tablets, sugar pills, capsules, pills and granules can be prepared by using coating and shell materials, such as enteric coatings and any other materials known in the art. They can contain an opaque agent. The release of the active compounds or compounds in the compositions can be released in a delayed mode in a given portion of the digestive tract. Examples of the embedding components include polymers and waxes. If necessary, the active compounds and one or more above excipients can form microcapsules.

Liquid dosage forms for oral administration include pharmaceutically acceptable emulsions, solutions, suspensions, syrups or tinctures. In addition to the active compounds, the liquid dosage forms may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

Besides these inert diluents, the composition may also contain additives such as wetting agents, emulsifiers, and suspending agent, sweetener, flavoring agents and perfume.

In addition to the active compounds, the suspension may contain suspending agent, for example, ethoxylated isooctadecanol, polyoxyethylene sorbitol and sorbitan esters, microcrystalline cellulose, methanol aluminum and agar, or the combination thereof.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

Compounds of the present invention can be administrated alone, or in combination with any other pharmaceutically acceptable compounds.

In the case of co-administration, the pharmaceutical composition can also include one or more other pharmaceutically acceptable compounds. One or more other pharmaceutically acceptable compounds may be used simultaneously, separately or sequentially with the compound of the present invention.

When the pharmaceutical compositions are used, a safe and effective amount of compound of the present invention is applied to a mammal (such as human) in need of, wherein the dose of administration is a pharmaceutically effective dose. For a person weighed 60 kg, the daily dose is usually 1-2000 mg, preferably 20-500 mg. Of course, the particular dose should also depend on various factors, such as the route of administration, patient healthy status, which are well within the skills of an experienced physician.

In the preferred embodiments of the present invention, the pharmaceutical compositions can be used:
(1) for the treatment of various tumors, including but not limited to melanoma (e.g. metastatic malignant melanoma), renal cancer (e.g. clear cell carcinoma), prostate cancer (e.g. hormone refractory prostate adenocarcinoma), breast cancer, colon cancer and lung cancer (e.g. non-small cell lung cancer), bone cancer, pancreatic cancer, skin cancer, head or neck cancer, skin or intraocular melanoma, uterine cancer, ovarian cancer, rectal cancer, anal cancer, gastrointestinal cancer, testicular cancer, uterine cancer, fallopian tube cancer, endometrial cancer, cervical cancer, vaginal cancer, vulva cancer, Hodgkin's disease, non-Hodgkin's lymphoma, esophageal cancer, small bowel cancer, endocrine system cancer, thyroid cancer, parathyroid cancer, adrenal cancer, soft tissue sarcoma, urethral cancer, penile cancer, chronic or acute leukemia (including acute myeloid leukemia, chronic myeloid leukemia, acute lymphoblastic leukemia, chronic lymphocytic leukemia), childhood solid tumors, lymphocytic lymphoma tumors, bladder cancer, kidney or ureter cancer, renal pelvis cancer, central nervous system (CNS) neoplasms/tumors, primary CNS lymphoma, tumor angiogenesis, spinal axis tumors, brainstem glioma, pituitary gland adenoma, Kaposi's sarcoma, epidermoid carcinoma, squamous cell carcinoma, T-cell lymphoma, environmentally induced cancers (including those induced by asbestos), and the combination thereof. Metastatic cancer, especially metastatic cancer that expresses PD-L1.
(2) in combination administration regimes, such as combined tumor chemotherapy, other tumor immunotherapeutics (small molecule compounds, antibodies, etc.), radiotherapy, tumor-targeted drugs, tumor vaccines, such as human papilloma virus (HPV), hepatitis virus (HBV and HCV) and Kaposi herpes sarcoma virus (KHSV)). It can be administered before, after, or simultaneously with the agent, or it can be co-administered with other known therapies.
(3) alone or in combination for the treatment of patients exposed to specific toxins or pathogens, which includes, but is not limited to, the treatment of various viruses, pathogenic bacteria, pathogenic fungi, pathogenic parasites, etc., e.g., infections established by pathogens, such as HIV, hepatitis virus (A, B, C), influenza virus, herpes virus, Giardia, malaria, Leishmania, staphylococcus aureus, pseudomonas aeruginosa..
(4) to induce therapeutic autoimmune response to treat patients with inappropriate accumulation of other autoantigens, such as amyloid deposits, including Aβ in Alzheimer's disease, cytokines such as TNFα and IgE .

The present invention will be further illustrated below with reference to the specific examples. It should be understood that these examples are only to illustrate the invention but not to limit the scope of the invention. The experimental methods with no specific conditions described in the following examples are generally performed under the conventional conditions, or according to the manufacturer's instructions. Unless indicated otherwise, parts and percentage are calculated by weight.

In each examples:
Analysis Method I
LCMS: Agilent 6110, UV detector: G1315D
Chromatography column: Xbridge C18 3.0 X 50 mm, 2.5 uM, column temperature 30 °C.
Mobile phase: A: H₂O (0.05% TFA), B: acetonitrile, gradient elution: 0-1 min 10% B, 1-8 min 10-95% B, 9 min 95% B

### Synthesis of Intermediate A:

### 2-3-Bromo-2-chlorohenl-2H-razolo3,4-bridine-5-carbaldehde

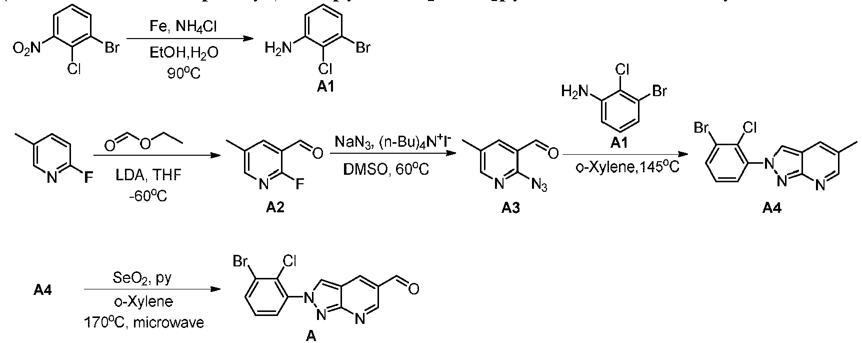

### 3-Bromo-2-chloroaniline

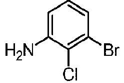

1-Bromo-2-chloro-3-nitrobenzene (5.0 g, 21 mmol) was dissolved in ethanol (100 mL) and water (10 mL), then iron powder (5.9 g, 105 mmol) and ammonium chloride (5.6 g, 105 mmol) were added to the solution at room temperature. The reaction mixture was stirred and reacted at 90 °C for two hours. The reaction mixture was filtered. The filter cake was washed three times with dichloromethane and the filtrate was concentrated to obtain a crude product. The crude product was dispersed in saturated aqueous sodium bicarbonate solution and extracted with dichloromethane. The organic phase was dried with anhydrous sodium sulfate, filtered and concentrated to obtain the target compound (4 g, 91%) as gray oil.
MS (ESI): m/z = 206.0, 208.0 [M+H]⁺.

### 2-Fluoro-5-methlnicotine aldehyde

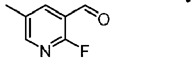

2-Fluoro-5-methylpyridine (20 g, 180 mmol) was dissolved in tetrahydrofuran (300 mL). Lithium diisopropylamide (2M, 135 mL, 270 mmol) was added to the solution under nitrogen atomsphere at -60 °C. After the reaction mixture was stirred for 30 minutes, ethyl formate (27 g, 360 mmol) was added. The mixture was kept at -60 °C and stirred for another hour. TLC showed that the reaction was completed; the reaction mixture was poured into aqueous hydrochloric acid, extracted with ethyl acetate. The organic phase was dried over anhydrous sodium sulfate, concentrated to obtain a crude product. The crude product was purified by silica gel column (petroleum ether/ethyl acetate=10/1 elution) to obtain the target compound (10.5 g, 42%) as a yellow solid.

### 2-Azido-5-methylnicotine aldehyde

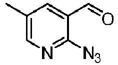

2-Fluoro-5-methylnicotine aldehyde (10.5 g, 75 mmol) and tetrabutylammonium iodide (2.8 g, 7.5 mmol) were dissolved in dimethyl sulfoxide (100 mL). Sodium azide (5.9 g, 90 mmol) was added to the solution at room temperature. The resulting mixture was stirred overnight at 60 °C, the reaction mixture was poured into water and filtered. The filter cake was washed three times with water, and dried to obtain the target compound as a yellow solid (7.1 g, 58%). MS (ESI): *m*/*z* = 163.0 [M+H]⁺.

### 2-(3-Bromo-2-chlorophenyl)-5-methyl-2H-pyrazolo[3,4-b]pyridine

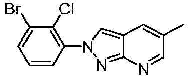

2-Azido-5-methylnicotine aldehyde (3 g, 19 mmol) and 3-bromo-2-chloroaniline (3.9 g, 19 mmol) were dissolved in o-xylene (100 mL), and the solution was stirred overnight at 145 °C. The reaction mixture was purified by silica gel column (dichloromethane/methanol=10/1 eluted) to obtain target compound (2.5 g, 42%) as a gray solid.
¹H NMR (400 MHz, CDCl₃) δ 8.65 (s, 1H), 8.23 (s, 1H), 7.85 (s, 1H), 7.78 (d, *J* = 8.0Hz, 1H), 7.69 (d, *J*= 8.0Hz, 1H), 7.32 (dd, *J*= 8.0, 8.0 Hz, 1H), 2.47 (s, 3H).

### 2-(3-Bromo-2-chlorophenyl)-2H-pyrazolo[3,4-b]pyridine-5-carbaldehyde

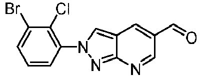

2-(3-Bromo-2-chlorophenyl)-5-methyl-2H-pyrazolo[3,4-b]pyridine (2.0 g, 6.19 mmol) and pyridine (1 mL, 12 mmol) were dissolved in o-xylene (10 mL). Selenium dioxide (6.7 g, 60 mmol) was added at room temperature, and the mixture was stirred under microwave at 170 °C for 16 hours. The reaction mixture was filtered. The filter cake was washed three times with ethyl acetate, and the filtrate was extracted with ethyl acetate. The organic phase was dried over anhydrous sodium sulfate, filtered and concentrated to obtain a crude product; the crude product was purified by prep-HPLC to obtain the target compound (60 mg) as an off-white solid.
¹H NMR (400 MHz, DMSO-*d*₆): *δ* (ppm) 10.14 (s, 1H), 9.23 (s, 1H), 9.14 (d, *J*=2Hz, 1H), 9.01 (d, *J*=2Hz, 1H), 8.10-8.08 (m, 1H), 7.87-7.84 (m, 1H), 7.58 (t, *J*=8Hz, 1H).
MS-ESI: m/z 338[M+H]⁺.

### Synthesis of Intermediate B:

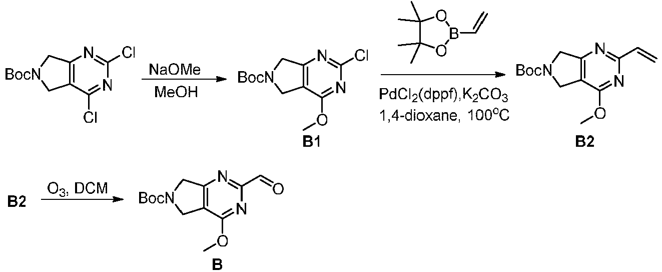

### Tert-Butyl 2-chloro-4-methoxy-5,7-dihydro-6H-pyrrolo[3,4-d]pyrimidine-6-carboxylate

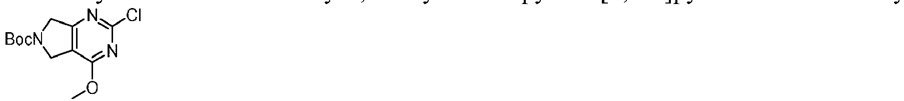

To the solution of tert-butyl 2,4-dichloro-5,7-dihydro-6H-pyrrolo[3,4-d]pyrimidine-6-carboxylate (3.0 g, 10.3 mmol) in dry methanol (50 mL), sodium methoxide (800 mg, 15.5 mmol) was added. The reaction mixture was reacted at room temperature for half an hour, quenched with water (100 mL), extracted with dichloromethane for three times. The combined organic phase was dried over anhydrous sodium sulfate, filtered, and concentrated; the crude product was purified by silica column to obtain the target compound (2.5 g, 85%) as a white solid.

### Tert-Butyl 4-methoxy-2-vinyl-5,7-dihydro-6H-pyrrolo[3,4-d]pyrimidine-6-carboxylate

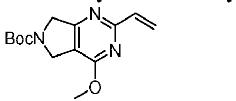

[1,1'-bis(diphenylphosphino)ferrocene]palladium dichloride (0.3 g, 0.4 mmol) was added to the mixture of compound B1 (2.5 g, 8.75 mmol), 4,4,5,5-tetramethyl-2-vinyl-1,3,2-dioxaborolane (2.02 g, 13.12 mmol) and potassium carbonate (2.42 g, 17.50 mmol) in 1,4-dioxane (50 mL) and water (5 mL), and then the reaction mixture was heated to 100 °C and reacted under nitrogen for 10 hours. The reaction solution was cooled, quenched with water (100 mL) and extracted three times with ethyl acetate. The organic phase was combined, dried over anhydrous sodium sulfate, filtered, and concentrated. The crude was purified by column to obtain the target compound (2.0 g, 82%). MS (ESI): m/z = 278.1 [M+H]⁺.

### Tert-Butyl 2-formyl-4-methoxy-5, 7-dihydro-6H-pyrrolo[3,4-d]pyrimidine-6-carboxylate

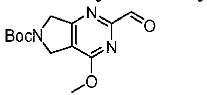

Ozone was bubbled into the solution of compound B2 (1 g, 3.61 mmol) in dichloromethane (50 mL); after ten minutes, the reaction mixture was quenched with saturated sodium thiosulfate solution (100 mL), and extracted with dichloromethane. The organic phase was washed three times with saturated brine, dried over anhydrous sodium sulfate, filtered, and concentrated, the crude product was purified by column with ethyl acetate and petroleum ether to obtain the target compound (0.4 g, 40%) as a white solid.
¹H NMR (400 MHz, CDCl₃): *δ* 10.03 (brs, 1H), 4.76-4.65 (m, 4H), 4.15 (s, 3H), 1.52 (s, 9H).

### Synthesis of Intermediate C:

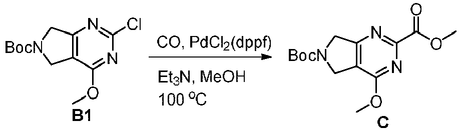

### 6-(Tert-butyl) 2-methyl 4-methoxy-5,7-dihydro-6H-pyrrolo[3,4-d]pyrimidine-2,6-dicarboxylate

A solution of compound B1 (0.5 g, 1.75 mmol) and triethylamine (0.5 g, 5.25 mmol) in methanol (10 mL) was added into an autoclave and heated to 100 °C under carbon monoxide (10 atm). After 10 hours, the reaction mixture was concentrated and the crude product was purified by flash chromatography (ethyl acetate and petroleum ether) to obtain the target compound (0.3 g, 55%) as a white solid.
¹H NMR (400 MHz, CDC13): δ 4.75-4.64 (m, 4H), 4.14 (s, 3H), 4.04 (s, 3H), 1.52 (s, 9H).

### Example 1:

### 2-(((2-(2-Chloro-[1,1'-biphenyl]-3-yl)-2H-pyrazolo[3,4-b]pyridin-5-yl)methyl)amino) ethane-1-ol

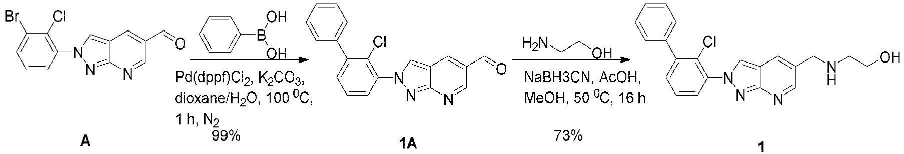

### 2-(2-Chloro-[1,1'-biphenyl]-3-yl)-2H-pyrazolo[3,4-b]pyridine-5-carbaldehyde.

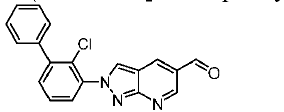

To the mixture of 2-(3-bromo-2-chlorophenyl)-2H-pyrazolo[3,4-b]pyridine-5-carbaldehyde (50 mg, 0.15 mmol), phenylboronic acid (37 mg, 0.30 mmol) in 1,4-dioxane (3 mL) and water (0.5 mL), [1,1'-bis(diphenylphosphino)ferrocene]palladium dichloride (11 mg, 0.015) and potassium carbonate (62 mg, 0.45 mmol) was added. The reaction mixture was heated to 100 °C and stirred for one hour under nitrogen. The reaction mixture was cooled down and filtered, washed twice with methanol, and concentrated. The residue was purified by normal phase chromatography (petroleum ether: ethyl acetate=50:1) to obtain the title compound 2-(2-chloro-[1,1'-biphenyl]-3-yl)-2H-pyrazolo[3,4-b]pyridine-5-carbaldehyde (50 mg, 99%) as a pale yellow solid.
MS (ESI): m/z = 334.0 [M+H]⁺.

### 2-(((2-(2-Chloro-[1,1'-biphenyl]-3-yl)-2H-pyrazolo[3,4-b]pyridin-5-yl)methyl)amino) ethane-1-ol

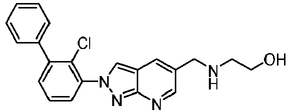

To the solution of example 1A (10 mg, 0.030 mmol), 2-aminoethane-1-ol (4 mg, 0.060 mmol) in methanol, a catalytic amount of acetic acid (1 drop) was added. The reaction solution was heated to 60 °C and stirred for half an hour. Sodium cyanoborohydride (4 mg, 0.030 mmol) was added, and the reaction solution was stirred overnight. The reaction mixture was cooled, filtered, concentrated and the residue was purified by prep-HPLC to obtain the target product (8 mg, 73%) as a white solid.
MS (ESI): m/z = 379.1 [M+H]⁺.
¹H NMR (400 MHz, DMSO-*d*₆) *δ* 8.75 (s, 1H), 8.65 (d, *J* = 2.1 Hz, 1H), 8.11 (d, *J* = 1.8 Hz, 1H), 7.73 (dd, *J=* 6.5, 3.0 Hz, 1H), 7.64-7.60 (m, 2H), 7.50-7.46 (m, 4H), 7.45-7.40 (m, 1H), 4.46 (t, *J* = 5.3 Hz, 1H), 3.80 (s, 2H), 3.45 (m, 2H), 2.56 (t, *J* = 5.8 Hz, 2H).

### Example 2:

### 1-((2-(2-Chloro-[1,1'-biphenyl]-3-yl)-2H-pyrazolo[3,4-b]pyridin-5-yl)methyl)piperidine -2-carboxylic acid

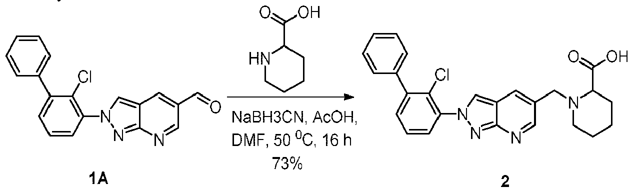

### 1-((2-(2-Chloro-[1,1'-biphenyl]-3-yl)-2H-pyrazolo[3,4-b]pyridin-5-yl)methyl)piperidine -2-carboxylic acid

A catalytic amount of acetic acid (1 drop) was added to the solution of example 1A (25 mg, 0.075 mmol), piperidine-2-carboxylic acid (19 mg, 0.15 mmol) in N,N-dimethylformamide (3 mL). The reaction solution was heated to 60 °C and stirred for half an hour, then sodium cyanoborohydride (14 mg, 0.23 mmol) was added. The reaction mixture was stirred overnight. The reaction mixture was filtered, concentrated, and the residue was purified by prep-HPLC to obtain the target product 1- ((2-(2-chloro-[1,1'-biphenyl]-3-yl)-2H-pyrazolo[3,4-b]pyridin-5-yl)methyl)piperidine-2 -carboxylic acid (8 mg, 73%) as a white solid.
MS (ESI): m/z = 447.1 [M+H]⁺.
¹H NMR (400 MHz, DMSO-*d*₆) δ 8.79 (s, 1H), 8.67 (d, *J* = 2.2 Hz, 1H), 8.12 (d, *J* = 1.9 Hz, 1H), 7.72 (dd, *J*= 6.4, 2.9 Hz, 1H), 7.66-7.60 (m, 2H), 7.51-7.46 (m, 3H), 7.45-7.40 (m, 1H), 3.93 (d, *J =* 13.4 Hz, 1H), 3.57 (d, *J=* 13.3 Hz, 1H), 3.16-3.10 (m, 1H), 2.91-2.83 (m, 1H), 2.27-2.18 (m, 1H), 1.84-1.62 (m, 2H), 1.60-1.40 (m, 3H), 1.40-1.24(m, 1H).

### Example 3:

### 2-(((7-Chloro-2-(2-methyl-[1,1'-biphenyl]-3-yl)-2H-indazol-5-yl)methyl)amino)ethane-1-alcohol

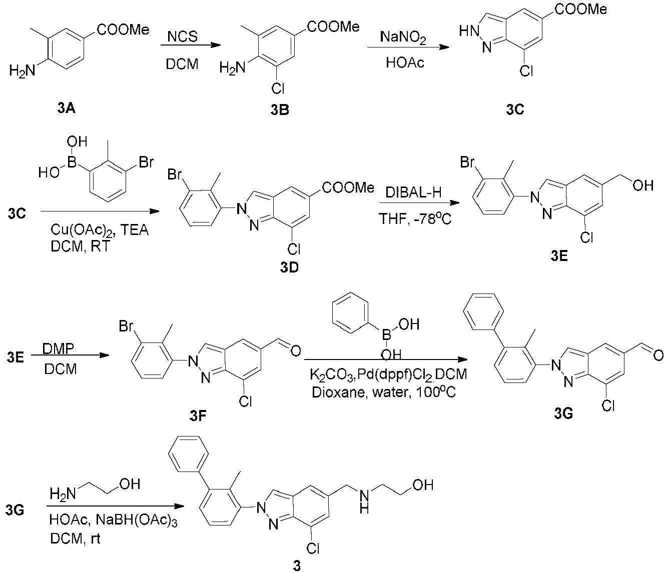

### Methyl 4-amino-3-chloro-5-methylbenzoate

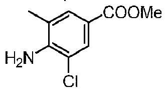

Methyl 4-amino-3-methyl benzoate (5.0 g, 30.3 mmol) and dry dichloromethane (50 mL) were added in a three-necked flask and stirred. N-chlorosuccinimide (4.85 g, 36.3 mmol) was added and the mixture was allowed to react overnight at room temperature. The reaction mixture was poured into water (100 mL), extracted three times with dichloromethane. The organic phase was combined, dried with anhydrous sodium sulfate, filtered, concentrated to dry, and purified by column to obtain the target compound (5.1 g, 82%) as a white solid.
¹H NMR (400 MHz, CDCl₃): δ 7.85 (s, 1H), 7.67 (s, 1H), 4.33 (brs, 2H), 3.86 (s, 3H), 2.22 (s, 3H).

### Methyl 7-chloro-2H-indazole-5-carboxylate

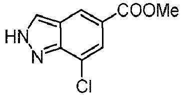

Compound 3B (200 mg, 1.0 mmol) and glacial acetic acid (5 mL) were added in a three-necked flask. An aqueous solution (1 mL) of sodium nitrite (84 mg, 1.2 mmol) was slowly added. The mixture was stirred at room temperature for 2 hours, quenched with water (100 mL), and extracted three times with ethyl acetate. The combined organic phases were washed with saturated sodium bicarbonate solution, dried over anhydrous sodium sulfate, filtered and concentrated. The crude product was purified by column to obtain the target compound (130 mg, 62%) as a red solid.
¹H NMR (400 MHz, CDCl₃): δ 10.60 (brs, 1H), 8.46 (s, 1H), 8.25 (s, 1H), 8.09 (s, 1H), 3.97 (s, 3H).

### Methyl 2-(3-bromo-2-methyphenyl)-7-chloro-2H-indazole-5-carboxlate

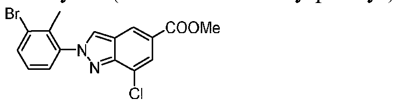

The mixture of compound 3C (210 mg, 1.0 mmol), (3-bromo-2-methylphenyl)boronic acid (430 mg, 2.0 mmol), triethylamine (550 mg, 5.0 mmol), copper acetate (363 mg, 2.0 mmol) and dry dichloromethane (20 mL) was added in a three-necked flask. The reaction mixture was stirred overnight at room temperature. The reaction solution was poured into ice water, and ammonia water was added to the aqueous phase, and extracted with ethyl acetate. The organic phase was combined and washed with saturated brine for three times, dried with anhydrous sodium sulfate, filtered and concentrated. The crude product was passed through the column with ethyl acetate and petroleum ether, and concentrated to obtain a white solid target compound (45 mg, 12%).

¹H NMR (400 MHz, CDCl₃): δ 8.50 (s, 1H), 8.29 (s, 1H), 8.02 (s, 1H), 7.74 (d, *J=* 8.0 Hz, 1H), 7.41 (d, *J* = 8.0 Hz, 1H), 7.25 (t, *J* = 8.0 Hz, 1H), 3.97 (s, 3H), 2.32 (s, 3H).

### (2-(3-Bromo-2-methylphenyl)-7-chloro-2H-indazol-5-yl)methanol

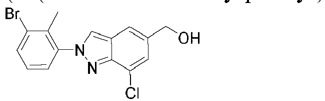

To a solution of compound 3D (160 mg, 0.42 mmol) in dry tetrahydrofuran (10 mL), a solution of diisobutylaluminum hydride (2 mL, 2.11 mmol) in toluene was added at -78 °C, then the reaction mixture was warmed to room temperature and stirred overnight. The reaction solution was quenched with ice water, extracted with ethyl acetate. The organic phase was combined, washed with saturated brine for three times, dried over anhydrous sodium sulfate, filtered and concentrated. The residue was purified by column with ethyl acetate and petroleum ether, and concentrated to dry to obtain the target compound as a white solid, which was directly used in the next reaction.
2-(3-Bromo-2-methylphenyl)-7-chloro-2H-indazole-5-carbaldehyde

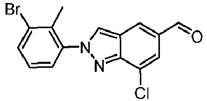

DMP (200 mg, 0.47 mmol) was added to a solution of compound 3E (138 mg, 0.39 mmol) in dichloromethane (10 mL) at room temperature and stirred overnight. The reaction mixture was poured into ice water and extracted with ethyl acetate. The combined organic phase was washed with saturated brine three times, dried over anhydrous sodium sulfate, filtered, and concentrated to dry. The residue was purified by column with ethyl acetate and petroleum ether to obtain the target compound as a white solid (100 mg, 73%).
¹H NMR (400 MHz, CDCl₃): *δ* 10.01 (s, 1H), 8.38 (s, 1H), 8.23 (s, 1H), 7.93 (s, 1H), 7.74 (d, *J =* 8.0 Hz, 1H), 7.41 (d, *J=* 8.0 Hz, 1H), 7.24 (t, *J* = 8.0 Hz, 1H), 2.45 (s, 3H).

### 7-Chloro-2-(2-methyl-[1,1'-biphenyl]-3-yl)-2H-indazole-5-carbaldehyde

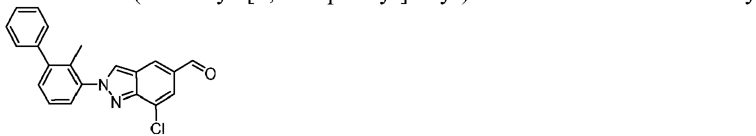

[1, 1'-Bis(diphenylphosphine)ferrocene]dichloride palladium dichloromethane complex (6mg, 0.007mmol) was added to a mixture of compound 3F (52mg, 0.15mmol), phenylboronic acid (27mg, 0.22mmol) and potassium carbonate (42mg, 0.3mmol) in 1,4-dioxane (5mL) and water (1mL). After being heated at 100 °C for 1 hour under nitrogen the reaction was filtered with celite and concentrated. The residue was purified by flash silica gel column chromatography (petroleum ether/ethyl acetate=2/1) to obtain the title compound (50mg, 96%) as a yellow solid.
MS (ESI): m/z = 347.1 [M+H]⁺.

### 2-(((7-Chloro-2-(2-methyl-[1,1'-biphenyl]-3-yl)-2H-indazol-5-yl)methyl)amino)ethane-1-alcohol

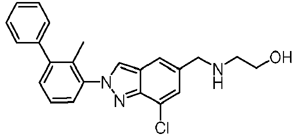

Acetic acid (1 drop) was added to the solution of compound 3G (15 mg, 0.043 mmol) and ethanolamine (8 mg, 0.13 mmol) in dichloromethane (5 mL). The solution was stirred at room temperature for 1 hour. After sodium triacetoxyborohydride (27 mg, 0.13 mmol) was added, the mixture was allowed to react at room temperature for 2 days. The reaction mixture was concentrated and the residue was separated and purified by reverse phase column (acetonitrile/formic acid aqueous solution (0.1%)) to obtain the title compound (8 mg, 47%) as a pink solid.
MS (ESI): m/z = 392.2 [M+H]⁺.
¹H NMR (400 MHz, DMSO-d6) δ 8.78 (s, 1H), 8.34 (s, 1H), 7.69 (s, 1H), 7.55-7.41 (m, 9H), 3.84 (s, 2H), 3.50 (t, *J=* 4 Hz, 2H), 2.62 (t, *J=* 4 Hz, 2H), 1.96 (s, 3H).

### Example 4

### (2S, 4S)-1-((7-chloro-2-(2-methyl-[1,1'-biphenyl]-3-yl)-2H-indazol-5-yl)methyl) -4-hydroxypyrrolidine-2-carboxylic acid

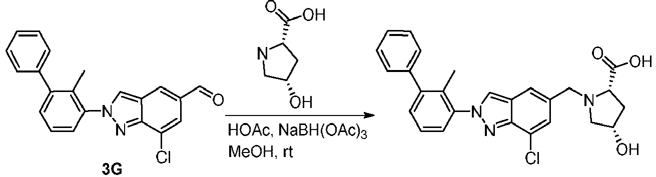

Acetic acid (1 drop) was added to the solution of compound 3G (45 mg, 0.13 mmol) and (2S,4S)-4-hydroxypyrrolidine-2-carboxylic acid (51 mg, 0.39 mmol) in methanol (5 mL). After the solution was stirred for 1 hour at room temperature, sodium cyanoborohydride (25 mg, 0.39 mmol) was added and the resulting mixture was reacted at room temperature overnight. After the reaction solution was concentrated, the residue was separated and purified by reverse phase column (acetonitrile/formic acid aqueous solution (0.1%)) to obtain the title compound (28 mg, 46%) as a white solid.
MS (ESI): m/z = 462.2 [M+H]⁺.
¹H NMR (400 MHz, DMSO-*d*₆) *δ* 8.80 (s, 1H), 7.69 (s, 1H), 7.55-7.53 (m, 2H), 7.42-7.48 (m, 3H), 7.46 (d, *J* = 2.0 Hz, 1H), 7.45-7.41 (m, 3H), 4.20 (s, 1H), 4.05 (d, *J* = 13.2 Hz, 1H), 3.63 (d, *J* = 13.0 Hz, 1H), 3.32-3.26 (m, 2H), 2.87 (d, *J=* 8.9 Hz, 1H), 2.65-2.61 (m, 1H), 2.39-2.32 (m, 1H), 1.96 (s, 3H), 1.81-1.75 (m, 1H).

### Example 5

### 2-(((6-(2-Chloro-[1,l'-biphenyl]-3-yl)-4-methoxy-6,7-dihydro-5H-pyrrolo[3,4-d)pyrimidin-2-yl)methyl)amino)ethane-1 -ol

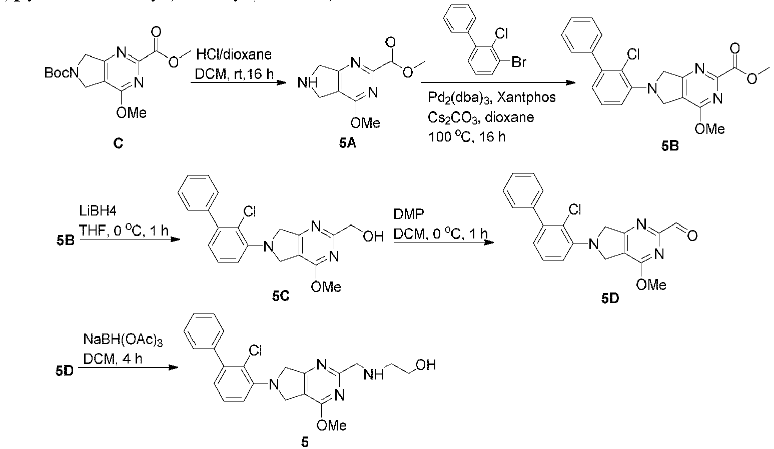

### Methyl 4-methoxy-6,7-dihydro-5H-pyrrolo[3,4-d]pyrimidine-2-carboxylate

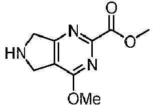

To a solution of intermediate C (1.08 g, 3.5 mmol) in dichloromethane (30 mL), a solution of hydrochloric acid in 1,4-dioxane (6 mL, 4M) was added. The reaction solution was stirred at room temperature for 16 hours; the reaction solution was concentrated, and the crude product was purified by prep-HPLC to obtain the target compound (810 mg, 100%) as a pale yellow solid.
MS (ESI): m/z = 210.1 [M+H]⁺.

### Methyl 6-(2-chloro-[1,1'-biphenyl]-3-yl)-4-methoxy-6,7-dihydro-5H-pyrrolo[3,4-d]pyrimidine -2-carboxylate

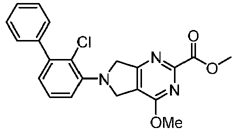

To the mixture of compound 5A (366 mg, 1.75 mmol), 3-bromo-2-chloro-1,1'-biphenyl (465 mg, 1.75 mmol) in 1,4-dioxane (3 mL), tris(dibenzylideneacetone)dipalladium(0) (82 mg, 0.09 mmol), 4,5-bisdiphenylphosphine-9,9-dimethylxanthene (76 mg, 0.18 mmol) and cesium carbonate (1.14 g, 3.50 mmol) were added. The reaction mixture was heated to 100 °C and stirred for 16 hours under the nitrogen; the reaction solution was cooled and concentrated, and the crude product was purified on a silica gel column (petroleum ether/ethyl acetate=10/15) to obtain the target compound 5B (100 mg, 14%) as a yellow solid.
MS (ESI): m/z = 396.1 [M+H]⁺.

### (6-(2-Chloro-[1,1'-biphenyl]-3-yl)-4-methoxy-6,7-dihydro-5H-pyrrolo[3,4-d]pyrimidine- 2-yl) methanol

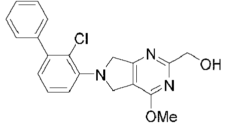

Lithium borohydride (0.1 mL, 0.20 mmol, 2 M solution in tetrahydrofuran) was added dropwise to a solution of compound 5B (80 mg, 0.20 mmol) in anhydrous tetrahydrofuran (5 mL), in an ice bath. The reaction solution was stirred in an ice bath for 15 minutes; quenched with water, and the crude product was separated and purified by flash normal phase (ethyl acetate/methanol=30/1) to obtain the target compound 5C (50 mg, 68%) as a yellow solid.
MS (ESI): *m*/*z* = 368.1 [M+H]⁺.

### 6-(2-Chloro-[1,1'-biphenyl]-3-yl)-4-methoxy-6,7-dihydro-5H-pyrrolo[3,4-d]pyrimidine-2 -formaldehyde

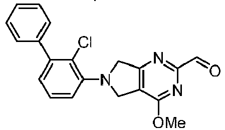

Dess-Martin oxidant (59 mg, 0.14 mmol) was added to the solution of 5C (50 mg, 0.14 mmol) in anhydrous dichloromethane (5 mL) in an ice bath, the reaction solution was stirred for 30 minutes and diluted with water (15 mL), then extracted three times with ethyl acetate (15 mL). The organic phase was dried and concentrated. The residue was purified by silica gel column (petroleum ether/ethyl acetate=10/15) to obtain the target compound 5D (20 mg, 39%) as a yellow solid.
MS (ESI): m/z = 366.1 [M+H]⁺.

### 2-(((6-(2-Chloro-[1,1'-biphenyl]-3-yl)-4-methoxy-6,7-dihydro-5H-pyrrolo[3,4-d)pyrimidin-2-yl)methyl)amino)ethane-1-ol

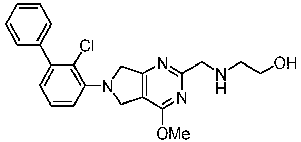

Catalytic amount of acetic acid (1 drop) was added to a solution of Example 5D (20 mg, 0.05 mmol), 2-aminoethane-l-ol (12 mg, 0.20 mmol) in anhydrous dichloromethane. After the reaction solution was stirred at room temperature for 2 hours, sodium triacetoxyborohydride (53 mg, 0.25 mmol) was added. The reaction solution was stirred for 2 hours and concentrated. The residue was purified by prep HPLC to obtain the target compound (8 mg, 40%), as a yellow solid.
MS (ESI): m/z = 411.4 [M+H]⁺.
¹H NMR (400 MHz, MeOH-d₄) δ 7.42-7.25 (m, 7H), 6.94 (dd, *J=* 6.7, 2.3 Hz, 1H), 4.75-4.69 (m, 4H), 4.07 (s, 3H), 3.96 (s, 2H), 3.70 (t, *J* = 5.4 Hz, 2H), 2.81 (t, *J* = 5.4 Hz, 2H).

### Example 6

### (S)-1-((6-(2-Chloro-[1,1'-biphenyl]-3-yl)-4-methoxy-6,7-dihydro-5H-pyrrolo[3 ,4-d)pyrimidin-2-yl)methyl)piperidine-2-carboxylic acid

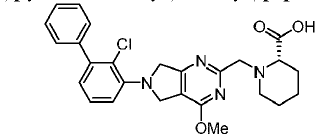

The target compound was prepared from compound 5D and (S)-piperidine-2-carboxylic acid by conditions similar to Example 5.
MS (ESI): m/z = 479.2 [M+H]⁺.
¹H NMR (400 MHz, MeOH-*d*₄) *δ* 8.46 (s, 1H), 7.43-7.25 (m, 7H), 6.96 (dd, *J=* 6.4, 2.5 Hz, 1H), 4.78-4.76 (m, 2H), 4.76-4.72 (m, 2H), 4.54 (brs, 1H), 4.40 (brs, 1H), 4.09 (s, 3H), 3.70-3.61 (m, 2H), 3.15-3.04 (m, 1H), 2.15 (brs, 1H), 2.02 (brs, 1H), 1.82-1.76 (m, 3H), 1.59 (brs, 1H).

### Example 7

### 2-(((7-Bromo-2-(2-methyl-[1,1'-biphenyl]-3-yl)-2H-indazol-5-yl)methyl)amino)ethane-1-alcohol

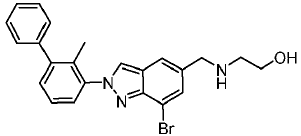

The target compound was obtained with the method similar to Example 3.
MS (ESI): m/z = 438.1 [M+H]⁺.
¹H NMR (400 MHz, CD3OD) δ 8.63 (s, 1H), 7.88 (s, 1H), 7.72 (d, *J*= 1.3 Hz, 1H), 7.48-7.42 (m, 5H), 7.42-7.32 (m, 3H), 4.15 (s, 2H), 3.76 (t, *J=* 6 Hz, 2H), 3.00 (t, *J* = 4 Hz, 2H), 1.96 (s, 3H), 1.92 (d, *J =* 5.2 Hz, 2H).

### Example 8

### 7-Methyl-2-(2-methyl-[1,1'-biphenyl]-3-yl)-2H-indazol-5-yl)methyl)amino)ethane-1-ol

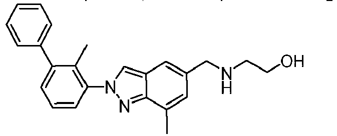

The target compound was obtained with the method similar to Example 3.
MS (ESI): m/z = 372.2 [M+H]⁺.
¹H NMR (400 MHz, CD₃OD) δ 8.53 (s, 1H), 8.45 (s, 1H), 7.71 (s, 1H), 7.49-7.40 (m, 5H), 7.40-7.33 (m, 3H), 7.20 (s, 1H), 4.15 (s, 2H), 3.77 (t, *J=* 4 Hz, 2H), 3.02 (t, *J* = 4 Hz, 2H), 2.61 (s, 3H), 1.95 (s, 3H).

### Example 9

### (((2-Hydroxyethyl)amino)methyl)-2-(2-methyl-[1,1'-biphenyl]-3-yl)-2H-indazole-7-carbonitrile

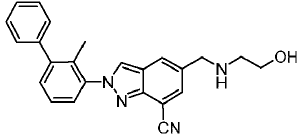

The target compound was obtained with the method similar to Example 3.
MS (ESI): m/z = 383.2 [M+H]⁺.
¹H NMR (400 MHz, CD₃OD) δ 8.69 (s, 1H), 8.51 (s, 1H), 8.17 (s, 1H), 7.96 (d, *J* = 1.4 Hz, 1H), 7.51-7.41 (m, 5H), 7.39-7.36 (m, 3H), 4.55 (s, 1H), 4.09 (s, 2H), 3.73 (t, *J* = 4 Hz, 2H), 2.90 (t, *J =* 4 Hz, 2H), 1.98 (s, 3H).

### Example 10

### 2-(((2-(2-Chloro-[1,1'-biphenyl]-3-yl)-7-methoxyisoindolin-5-yl)methyl)amino)ethane-1-ol

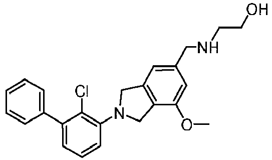

The target compound was obtained with the method similar to Example 5.
MS (ESI): m/z = 409.4 [M+H]⁺.
¹H NMR (400 MHz, MeOD) *δ* 7.42-7.31 (m, 5H), 7.28-7.18 (m, 2H), 6.92-6.87 (m, 3H), 4.74-4.72 (m, 2H), 4.69-4.66 (m, 2H), 3.87 (s, 3H), 3.85 (s, 2H), 3.68 (t, *J=* 5.5 Hz, 2H), 2.78 (t, *J* = 5.5 Hz, 2H).

### Test Example 1: Detect the inhibitory effect of the compound to the binding of PD-1/PD-L1 protein

The PD-1/PD-L1 homogeneous time-resolved fluorescence method is used to detect the binding ability of the compound with PD-L1.

PD-1/PD-L1 binding assay kit (Cisbio, Cat#63ADK000CPDEC) was chosen, which contains two proteins Tag 1-PD-L1 and Tag 2-PD-1, and two antibodies Anti-Tag 1-Eu³⁺ and Anti-Tag2- XL 665. The principle of detection was: Anti-tag 1-Eu³⁺ was used as the donor of HTRF, and Anti-Tag2-XL 665 was used as the acceptor of HTRF. When Tag 1-PD-L1 interacts with Tag 2-PD-1, the added HTRF donor and the acceptor are close to each other. After the donor has received the excitation energy, part of the energy was transferred to the acceptor, thus generating 665 nm emission light. When the addition of the compound blocked the PD-1/PD-L1 interaction, only 620 nm emission light was generated. The inhibitory effect of the compound can be determined by comparing the ratio of 665 nm/620 nm. Tag 1-PD-L1 was diluted with Diluent buffer (cat# 62DLBDDF) to a working concentration of 10 nM, Tag 2-PD-1 was diluted with Diluent buffer to a working concentration of 500 nM, Anti-Tag 1-Eu³⁺ was diluted with detection buffer (cat# 62DB1FDG) at a ratio of 1:100, Anti-Tag2-XL 665 was diluted with detection buffer at a ratio of 1:20, and the compound to be detected was diluted with diluent buffer to a final concentration of 2X. Compound (2 µL) was added to each well of a 384-well plate, then Tag 1-PD-L1 (4 µL) and Tag 2-PD-1 (4 µL) were added successively. The mixture was incubated at room temperature for 15 minutes. Then Anti-Tagl-Eu³⁺ (5 µL) and Anti-Tag2-XL 665 (5 µL) were added, and the mixture was incubated overnight at room temperature. BioTek Synergy™ Neo2 multifunctional microplate reader was used to detect so as to provide the 665 nm/620 nm ratio. PrismGraphd 5.02 was used to fit the IC₅₀ curve.

**Table 1 IC₅₀ of some compounds of the present invention**

| Compound number | PD-L1 IC₅₀ |
|---|---|
| 1 | B |
| 2 | N/T |
| 3 | N/T |
| 4 | N/T |
| 5 | A |
| 6 | A |
| 7 | N/T |
| 8 | N/T |
| 9 | B |
| 10 | A |

| | |
|---|---|
| A represents IC₅₀ of less than 100 nM; B represents IC₅₀ of 100nM to 1 uM; C represents IC₅₀ of greater than 1 uM; | |

The results show that the compound of the present invention can effectively inhibit the binding of PD-1/PD-L1 at different concentrations. Therefore, it can be used in the treatment of diseases related to the PD-1/PD-L1 interaction.

### Test example 2: Cellular NFAT reporter gene assay

Two types of cells (PD-1 effector cells and PD-L1 aAPC/CHO-K1 cells) were required in the cellular assay of PD-1/PD-L1. PD-1 effector cells express human PD-1 protein and luciferase report gene driven by NFAT, while PD-L1 aAPC/CHO-K1 cells express PD-L1 protein and anti-CD3 antibody. When these two kinds of cells were co-cultured, the interaction of PD-1/PD-L1 would inhibit the signal transmission from TCR to NFAT-RE and interrupt the fluorescence signal mediated by NFAT-RE. When the inhibitor of PD-1 or PD-L1 was added, the interaction of PD-1/PD-L1 was blocked, thus alleviating the inhibitory signal of the TCR to NFAT-RE pathway and enhancing fluorescence signal. The blocking effect of inhibitors was determined by the strength of fluorescence signals.

On the first day of the experiment, the recovered PD-L1 aAPC/CHO-K1 cells were trypsinized. After centrifugation, the concentration was adjusted to 2.5^{∗}10⁵/mL with the culture medium (90% Ham's F-12/10% FBS). Cells were plated in a 384-well plate at an amount of 40 µL, 1^{∗}10⁴ cells per well and placed in an incubator for overnight culture. The next day, the compound to be detected was diluted to 2 times of the required detection concentration with the detection buffer (99% RPMI1640/1% FBS) in gradient. PD-1 cells were centrifugated and adjusted to 6.25^{∗}10⁵/ mL using the detection buffer. The medium in the overnight cultured 384-well plate was discarded, and 20 µL of the diluted compound was added to each well, and 20 µL of PD-1 cells were added. After the plate was incubated in the cell culture incubator for 6 hours, 20 µL of Bio-Glo reagent was added to each well (Promega, cat#G7940). The plate was read with a multi-function microplate reader after 10 minutes. A negative control (only add cells, no compound), and a blank control (only add detection buffer) need to be set for each plate. Prism5 was used to analyze the inhibitory activity of the compound according to the fluorescence value.

The results show that the compound of the present invention can effectively block the interaction of PD-1/PD-L1, and the half-active inhibitory concentration is equivalent to or better than that of the PD-1 inhibitors in the clinical stage.

### Test example 3: Human PBMC functional assays

Two kinds of cells (human PBMC cells and PD-L1 aAPC/CHO-K1 cells) were required in human PBMC functional assay (CHO-K1 cells express full-length human PD-L1 protein and anti-hCD3 antibody). When the two kinds of cells were co-cultured, the anti-hCD3 antibody expressed by aAPC cells binds to CD3 on the surface of PBMC to stimulate PBMC activation and proliferation, but the interaction of PD-1/PD-L1 would inhibit TCR signal transmission. When PD-1 or PD-L1 inhibitors was added, the interaction of PD-1/PD-L1 was blocked, thus releasing the signal to the TCR pathway. The blocking effect of inhibitors was evaluated by detecting the changes of cell surface markers CD25, PD-1 and intracellular cytokine IFNγ after PBMC activation.

On the first day of the experiment, the recovered PD-L1 aAPC/CHO-K1 cells and PD-L1 Negative cells were trypsinized, and the cell concentration was adjusted to 2^{∗}10⁵/mL with medium (90% F12+10% FBS) after centrifugation. Cells were plated in a 96-well plate at 100 µL, 2^{∗}10⁴ cells per well and placed in an incubator for overnight culture. The next day, the cells were treated with a medium containing 10 µg/mL Mitomycin C. The plate was placed under 37°C for 4 hours and washed three times with PBS. The compound to be tested was prepared and diluted in gradient to 2 times of the required detection concentration. PBMC cells were recovered and adjusted to a concentration of 1^{∗}10⁶/mL according to the operating requirements. Compound in solution (50 µL) was added to the plate, and then 50 µL PBMC cells were added to the plate. After three days of incubation in a cell incubator, the cell supernatant was collected to detect the change of cytokine IFNγ by ELISA. Cells are collected for flow cytometry to detect changes in CD25 and PD-1.

The results show that the compounds of the present invention can effectively block the interaction of PD-1/PD-L1, and the half-active inhibitory concentration is equivalent to or better than that of the PD-1 inhibitors in the clinical phase.

### Test Example 4: In vivo drug efficacy study of the small molecule inhibitors of the present invention in treating tumors

A mouse model of subcutaneously inoculated tumors was established to examine the in vivo inhibitory effects of these compounds on tumor growth. The method was presented as follows: after the cultured specific tumor cells was trypsinized, the cells were collected by centrifugation, washed twice with sterile saline and counted. The cells were adjusted to the required concentration with saline. 0.2 mL of cell suspension was subcutaneously inoculated into C57BL/6 or Balb/c immunocompetent mice. After inoculation, the tumor growth was observed until becoming a specific volume. Animals were randomly grouped (6-7 animals in each group) and administered after weighing. The tested compound was administered once a day. The grouping includes: vehicle group, control anti-PD-Ll antibody group, testing compound groups. Mice were tested for tumor growth every week for about 6 weeks. After the tumor volume reached the tumor endpoint, the mice were weighed and euthanized. The tumor tissue, spleen tissue and blood samples were taken. Then the tumor inhibition rate was calculated, the immune cell composition in the tumor, spleen and blood samples was detected, and the immunomodulatory activity of the test compound was calculated.

The results show that the compounds of the present invention can effectively inhibit tumor growth in tumor-bearing mice, and inhibitory effect thereof is equivalent to or better than that of PD-1 inhibitors in the clinical phase.

### Test Example 5: In vivo drug efficacy study of the small molecule inhibitors of the present invention combined with chemotherapeutics in treating tumors

A mouse model of subcutaneously inoculated tumors was established to test the in vivo inhibitory effects of these compounds on tumor growth. The method was presented as follows: after the cultured specific tumor cells was trypsinized, the cells were collected by centrifugation, washed twice with sterile saline and counted. The cells were adjusted to the required concentration with saline. 0.2 mL of cell suspension was subcutaneously inoculated into C57BL/6 or Balb/c immunocompetent mice. After inoculation, the tumor growth was observed until becoming a specific volume. Animals were randomly grouped (6-7 animals in each group) and administered after weighing. The tested compound and the combination drug bevacizumab/ Carboplatin/ Paclitaxel/ Pemetrexed were administered in accordance with the combination regimen. The grouping includes: vehicle group, tested compound combined with chemotherapeutics group, chemotherapeutics group, and tested compound group. The grouping includes: vehicle group, control anti-PD-Ll antibody group, test compound groups. Mice were tested for tumor growth every week for about 6 weeks. After the tumor volume reached the tumor endpoint, the mice were weighed and euthanized. The tumor tissue, spleen tissue and blood samples were taken. Then the tumor inhibition rate was calculated, the immune cell composition in the tumor, spleen and blood samples was detected, and the immunomodulatory activity of the test compound and the combination drug was calculated.

The results show that the compounds of the present invention combined with chemotherapeutics can inhibit tumor growth in tumor-bearing mice more effectively, and inhibitory effect thereof is better than that of chemotherapeutics alone.

### Test Example 6: In vivo drug efficacy study of the small molecule inhibitors of the present invention combined with immunomodulator in treating tumors

A mouse model of subcutaneously inoculated tumors was established to test the in vivo inhibitory effects of these compounds on tumor growth. The method was presented as follows: after the cultured specific tumor cells was trypsinized, the cells were collected by centrifugation, washed twice with sterile saline and counted. The cells were adjusted to the required concentration with saline. 0.2 mL of cell suspension was subcutaneously inoculated into C57BL/6 or Balb/c immunocompetent mice. After inoculation, the tumor growth was observed until becoming a specific volume. Animals were randomly grouped (6-7 animals in each group) and administered after weighing. The testing compound and the combination drug Nivolumab/Ipilimumab were administered in accordance with the combination regimen. The grouping includes: vehicle group, testing compound combined with immunomodulator group, immunomodulator group, and testing compound group. Mice were tested for tumor growth every week for about 6 weeks. After the tumor volume reached the tumor endpoint, the mice were weighed and euthanized. The tumor tissue, spleen tissue and blood samples were taken. Then the tumor inhibition rate was calculated, the immune cell composition in the tumor, spleen and blood samples was detected, and the immunomodulatory activity of the test compound and the combination drug was calculated.

The results show that the compounds of the present invention combined with immunomodulatory drugs can inhibit tumor growth in tumor-bearing mice more effectively, and inhibitory effect thereof is better than that of immunomodulatory drugs alone.

### Test Example 7: The pharmacokinetic study of the small molecule inhibitors of the present invention in mice

The tested compound was administered to ICR mice intravenously (IV) and orally (PO) separately. Blood samples were collected at different time points. The concentration of test compound in the mouse plasma was determined by LC-MS/MS and related parameters were calculated. The details were as follows: the required amount of the test article was dissolved in 5% DMSO+10% Soluto1+85% water for injection to prepare a solution of the required concentration for intravenous or oral administration. The animals were about 6-8 weeks old at the start of the dosing experiment. Blood collection time points for intravenous: 0.083 h, 0.25 h, 0.5 h, 1 h, 2 h, 4 h, 8 h and 24 h after administration. Blood collection time points for oral: 0.25 h, 0.5 h, 1 h, 2 h, 4 h, 6 h, 8 h and 24 h after administration. Biological sample analysis methods and sample detection methods were established. The drug blood concentration at different time points were used to calculate the pharmacokinetic parameters with Phoenix WinNonlin 7.0 software, such as AUC₍₀₋ₜ₎, AUC_{(0-∞)}, T_{1/2}, Cmax, Tₘₐₓ and MRT, etc.

The results show that the compounds of the present invention show excellent pharmacokinetic properties.

### Test Example 8: The pharmacokinetic study of the small molecule inhibitors of the present invention in rat

The test compound was administered to SD rat intravenously (IV) and orally (PO) separately. Blood samples were collected at different time points. The concentration of test compound in the rat plasma was determined by LC-MS/MS and related parameters were calculated. The details were as follows: the required amount of the test article was dissolved in 5% DMSO+10% Solutol+85% water for injection to prepare a solution of the required concentration for intravenous or oral administration. The animals were about 6-8 weeks old at the start of the dosing experiment. Blood collection time points for intravenous: 0.083 h, 0.25 h, 0.5 h, 1 h, 2 h, 4 h, 8 h and 24 h after administration. Blood collection time points for oral: 0.25 h, 0.5 h, 1 h, 2 h, 4 h, 6 h, 8 h and 24 h after administration. Biological sample analysis methods and sample detection methods were established. The drug blood concentration at different time points were used to calculate the pharmacokinetic parameters with Phoenix WinNonlin 7.0 software, such as AUC₍₀₋ₜ₎, AUC_{(0-∞)}, T_{1/2}, Cmax, Tₘₐₓ and MRT, etc.

The results show that the compounds of the present invention show excellent pharmacokinetic properties.

All literatures mentioned in the present application are incorporated herein by reference, as though each one is individually incorporated by reference. Additionally, it should be understood that after reading the above teachings, those skilled in the art can make various changes and modifications to the present invention. These equivalents also fall within the scope defined by the appended claims.

## Claims

1. A compound according to Formula I, or stereoisomers or tautomers thereof, or pharmaceutically acceptable salts, hydrates or solvates thereof: 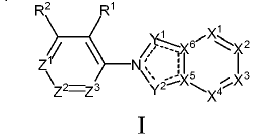
X¹, X², X³ and X⁴ are each independently selected from N and CR³;
X⁵ and X⁶ are each independently selected from N and C;
Y¹ and Y² are each independently N, C, C=O, S(=O), S(=O)₂, O or S;
Z¹, Z² and Z³ are each independently N or CR⁴;
R¹ is selected from H, halogen, CN, substituted or unsubstituted C₁-C₆ alkyl, substituted or unsubstituted C₁-C₆ alkoxyl;
R² is selected from the group consisting of substituted or unsubstituted C₆-C₁₀ aryl, substituted or unsubstituted 5-10 membered heteroaryl having 1-3 heteroatoms selected from the group consisting of N, S and O, and substituted or unsubstituted 5-10 membered heterocyclyl having 1-3 heteroatoms selected from the group consisting of N, S and O; and one or more H on R² are substituted by R⁵;
R³ is selected from H, substituted or unsubstituted C₁-C₆ alkyl, substituted or unsubstituted C₁-C₆ alkoxyl, 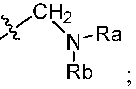 and at least one R³ is 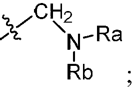
Ra and Rb are each independently selected from H, -(C=O)-substituted or unsubstituted C₁-C₈ alkyl, substituted or unsubstituted C₁-C₈ alkyl, substituted or unsubstituted C₂-C₆ alkenyl, substituted or unsubstituted C₂-C₆ alkynyl, substituted or unsubstituted C₁-C₈ alkylamino, substituted or unsubstituted C₁-C₈ alkoxyl, substituted or unsubstituted C₃-C₁₀ cycloalkyl, substituted or unsubstituted 3-10 membered heterocyclyl having 1-3 heteroatoms selected from the group consisting of N, S and O, substituted or unsubstituted C₆-C₁₀ aryl, substituted or unsubstituted 5-10 membered heteroaryl with 1-3 heteroatoms selected from the group consisting of N, S and O, or substituted or unsubstituted 5-10 membered heterocyclyl with 1-3 heteroatoms selected from the group consisting of N, S and O; or
Ra and Rb and adjacent N atoms form a substituted or unsubstituted 5-10 membered heterocyclyl with 1-3 heteroatoms selected from the group consisting of N, S and O;
R⁴ is selected from the group consisting of H, halogen, CN, substituted or unsubstituted C₁-C₆ alkyl, and substituted or unsubstituted C₁-C₆ alkoxyl;
R⁵ is selected from the group consisting of H, CN, halogen, substituted or unsubstituted-L₁-L₂(C₁-C₈ alkyl)-O-(C₁-C₈ alkylene)-(substituted or unsubstituted 5 -7 membered heteroaryl), substituted or unsubstituted -L₁-L₂(C₁-C₈ alkyl)-O-(C₁-C₈ alkylene)-N(Ra)(Rb), -O-substituted or unsubstituted -(C₁-C₈ alkylene alkyl)-O-(C₁-C₈ alkylene)-N(Ra)(Rb), or - NH-C(=O)-(substituted or unsubstituted 5-7 membered heteroaryl); wherein L₁ and L₂ are each independently selected from the group consisting of none, substituted or unsubstituted C₁-C₈ alkylene, -NH-C(=O)-NH-, -C (=O)-NH-, -O-, -S- or -NH-; or two R⁵ and connected carbon atoms form a group which is substituted or unsubstituted 5-7 membered heteroaryl, substituted or unsubstituted 5-7 membered heterocyclyl;
unless otherwise specified, "substituted" refers to being substituted by one or more (for example, 2, 3, 4, etc.) substituents selected from the group consisting of carboxyl, halogen, C₁-C₆ alkoxyl, halogenated C₁-C₆ alkoxyl, C₃-C₈ cycloalkyl, halogenated C₃-C₈ cycloalkyl, methyl sulfone group, -S(=O)₂NH₂, oxo(= O), -CN, hydroxyl, -NH₂, C₁-C₆ amine, C₁-C₆ amide -C=ON(Rc)₂ or -NH-C(=O)(Rc), Rc is H or C₁-C₅ alkyl), 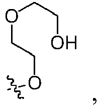 or substituted or unsubstituted groups selected from the group consisting of C₁-C₆ alkyl, C₆-C₁₀ aryl, 5-10 membered heteroaryl having 1-3 heteroatoms selected from N, S and O, -(CH₂)-C₆-C₁₀ aryl, -(CH₂)-(5-10 membered heteroaryl with 1-3 heteroatoms selected from N, S and O), and the substituent is selected from the group consisting of halogen, C₁-C₆ alkyl, C₁-C₆ alkoxyl, oxo, -CN, -OH, C₆-C₁₀ aryl, 5-10 membered heteroaryl with 1-3 heteroatoms selected from N, S and O; 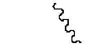
is the attachment site of the group;
each 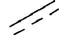 is a single bond or a double bond independently;
with the proviso that the compound of Formula I has a chemically stable structure.

2. The compound of claim 1, stereoisomers or tautomers thereof, or pharmaceutically acceptable salts, hydrates or solvates thereof, wherein the compound has a structure according to Formula II-1 or Formula II-2:

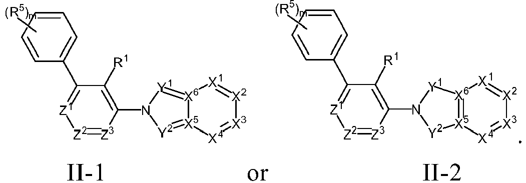

3. The compound of claim 1, wherein 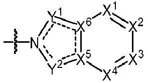 has a structure selected from the group consisting of

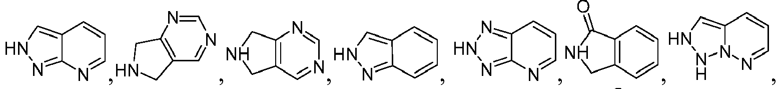

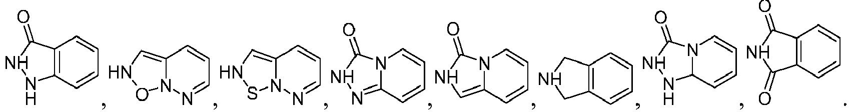

4. The compound of claim 1, wherein R³ is -CH₂-R, while the R is selected from the group consisting of

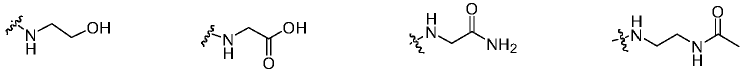

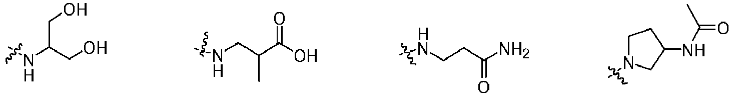

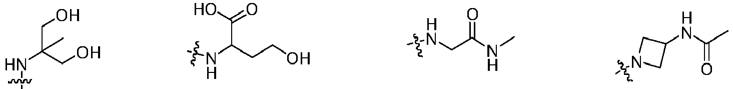

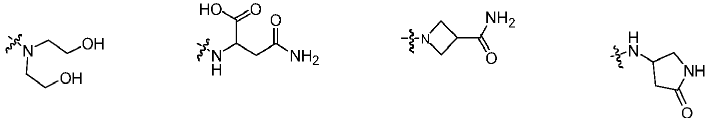

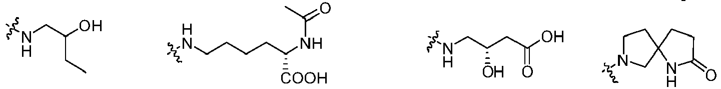

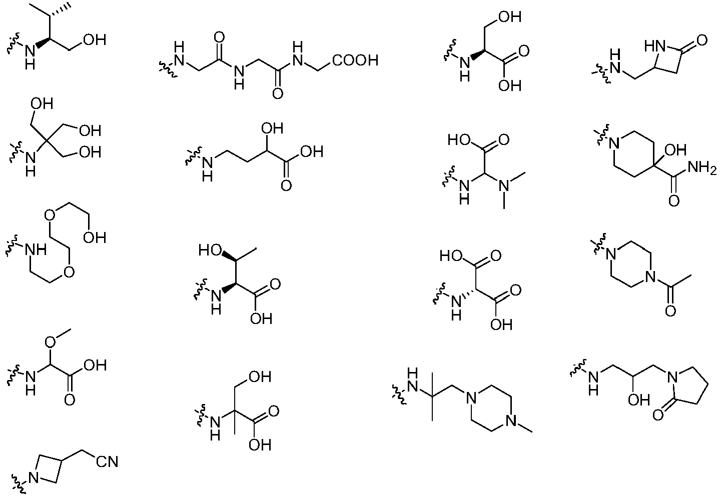

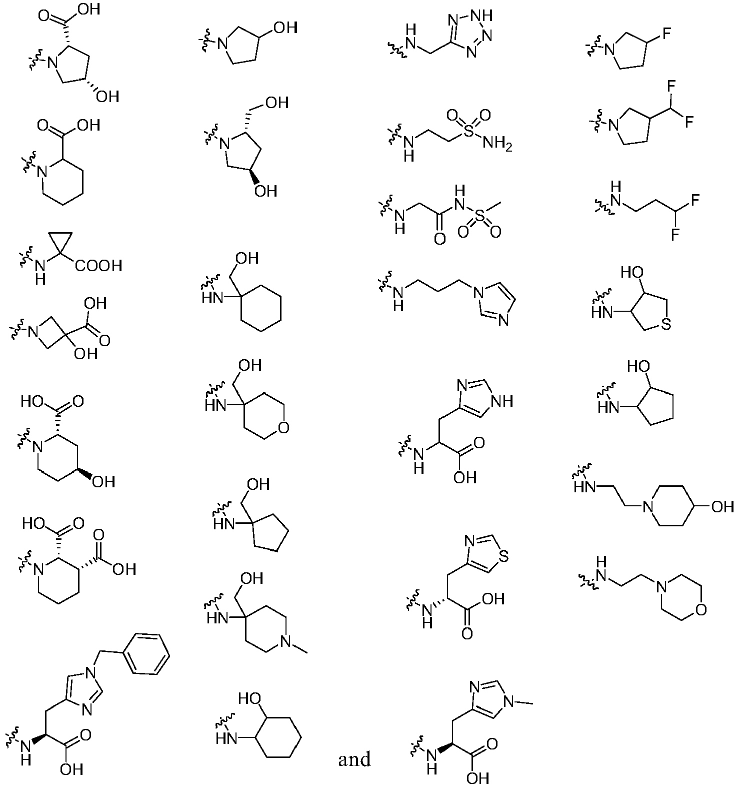

5. The compound of claim 1, wherein R⁵ is selected from the group consisting of H, Me, Cl and CN, or the group selected from the group consisting of: 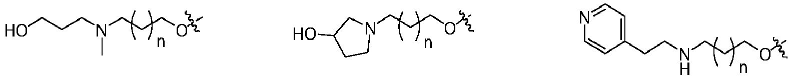 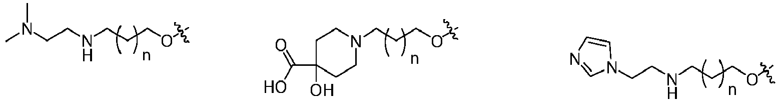 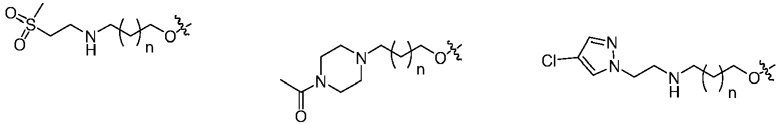 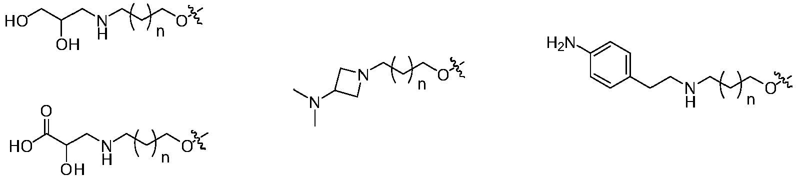 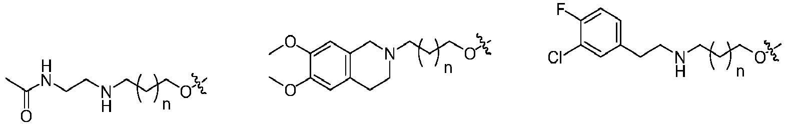 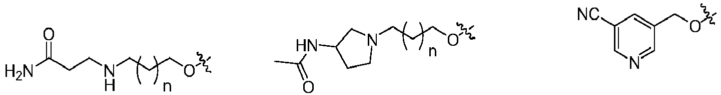 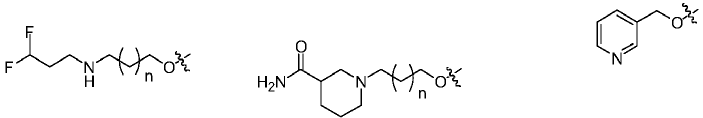 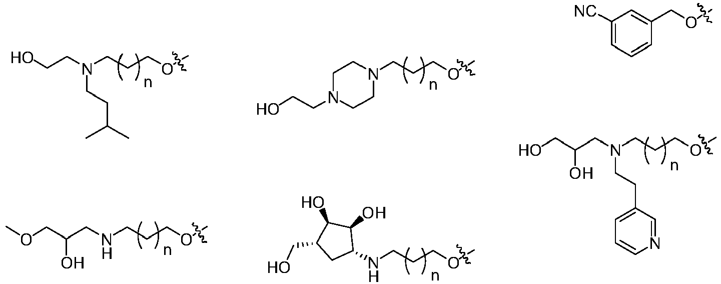 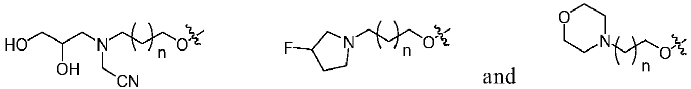 wherein, n=1-2.

6. The compound of claim 1, wherein the compound is selected from the group consisting of:

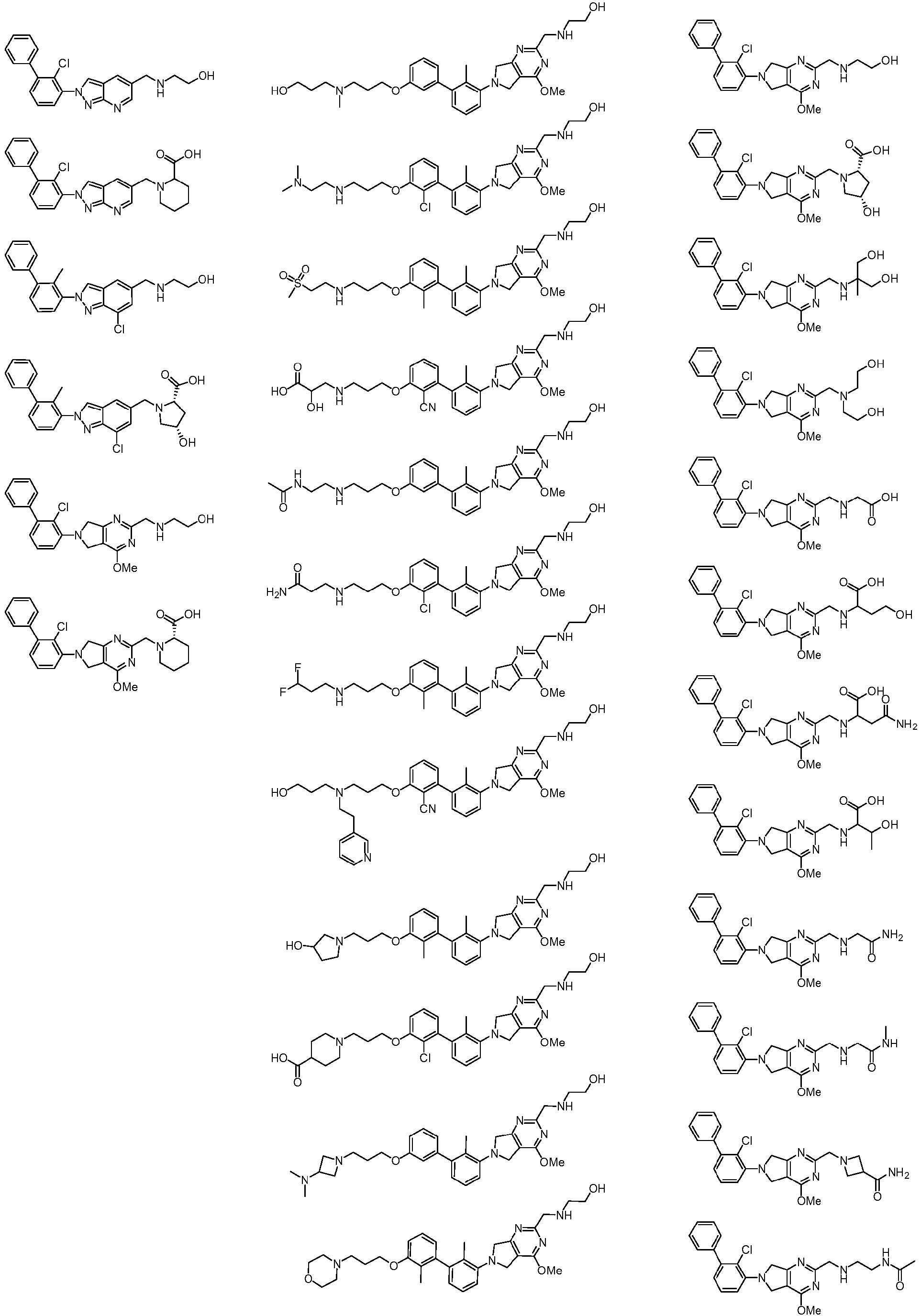

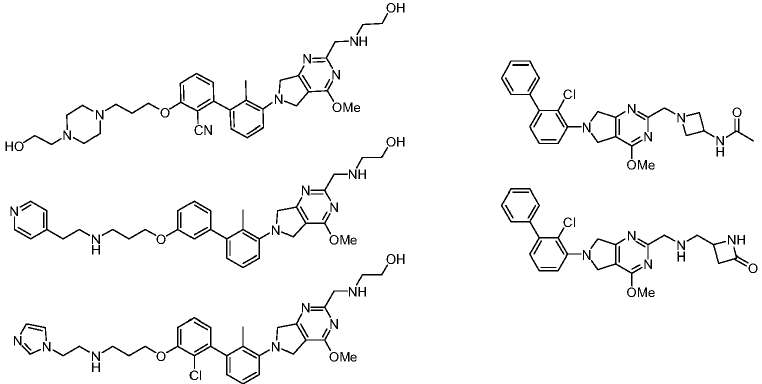

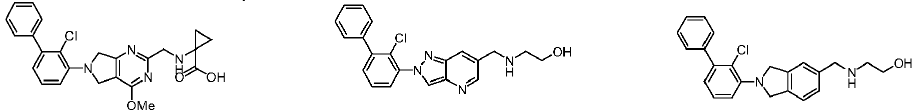

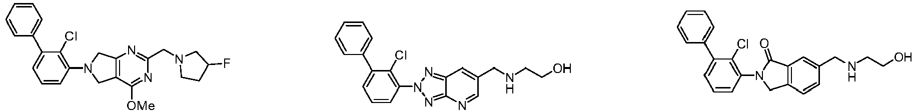

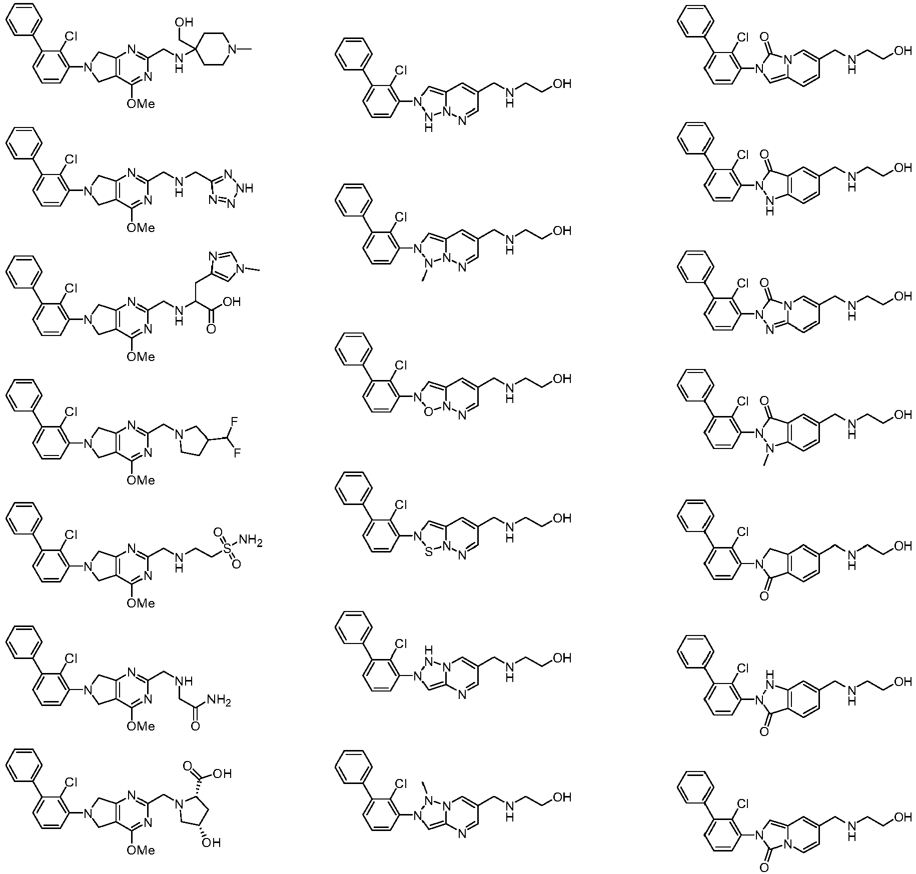

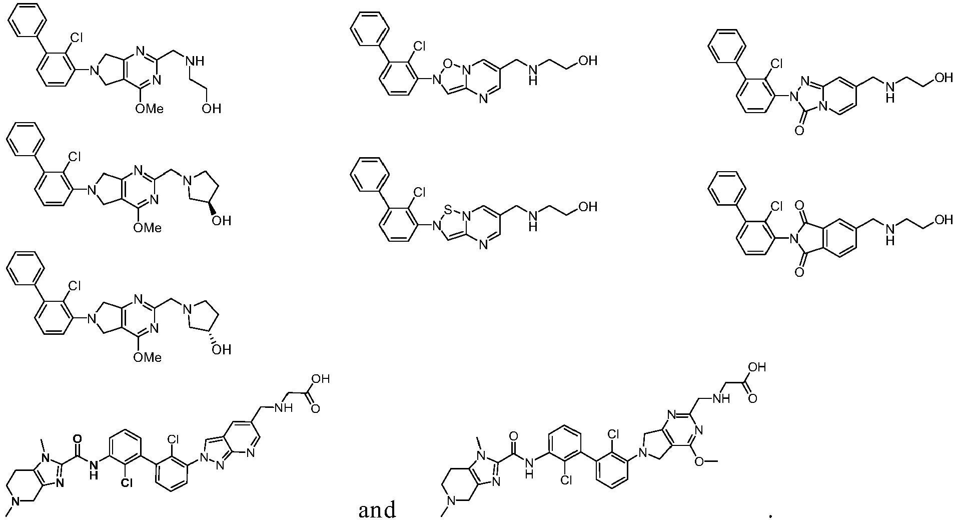

7. A pharmaceutical composition, comprising (1) the compounds of claim 1 or stereoisomers or tautomers thereof, or pharmaceutically acceptable salts, hydrates or solvates thereof; and (2) a pharmaceutically acceptable carrier.

8. The use of the compounds of claim 1 or stereoisomers or tautomers thereof, or pharmaceutically acceptable salts, hydrates or solvates thereof, or the pharmaceutical composition of claim 7, wherein it is used for preparation of pharmaceutical compositions for preventing and/or treating diseases related to the activity or expression of PD-1/PD-L1.

9. The use of claim 8, wherein the diseases are selected from the group consisting of tumors, pathogen infections, and diseases related to autoimmune responses.

10. A PD-1/PD-L1 inhibitor, wherein the inhibitor comprises the compound of claim 1, or stereoisomers or tautomers thereof, or pharmaceutically acceptable salts, hydrates or solvates thereof.
